# EUROPEAN PATENT SPECIFICATION

(11) **EP 4 431 022 B1**
(45) Date of publication and mention of the grant of the patent: **26.11.2025**
(21) Application number: 24162988.0
(22) Date of filing: 12.03.2024
(51) Int. Cl.: A61B 6/06, A61B 6/46, G21K 1/02

(54) **X-RAY DIAGNOSTIC APPARATUS AND METHOD OF OPERATING IRRADIATION FIELD**
RÖNTGENDIAGNOSTIKGERÄT UND VERFAHREN ZUM BETRIEB EINES BESTRAHLUNGSFELDES
APPAREIL DE DIAGNOSTIC À RAYONS X ET PROCÉDÉ DE FONCTIONNEMENT DE CHAMP D'IRRADIATION

(30) Priority: 13.03.2023 JP 2023038730; 18.01.2024 JP 2024006314
(43) Date of publication of application: 18.09.2024
(73) Proprietor: Canon Medical Systems Corporation, Tochigi (JP)
(72) Inventor: YAMADA, Naoki, Otawara-shi (JP); OOHASHI, Toshikatsu, Otawara-shi (JP); TAKAHASHI, Daisuke, Otawara-shi, 324-0036 (JP); INAGAKI, Takahiro, Otawara-shi (JP); OKUMURA, Yusuke, Otawara-shi (JP); MOROMIZATO, Rui, Otawara-shi, 324-0036 (JP); NISHIZUKA, Seiichi, Otawara-shi (JP)
(74) Representative: Marks & Clerk LLP

(56) References cited:
- EP-B1- 2 754 394
- US-A1- 2016 051 210
- US-A1- 2023 000 449

## Description

### FIELD

Embodiments disclosed in the present specification and drawings relate to an X-ray diagnostic apparatus and a method of operating an irradiation field.

### BACKGROUND

Conventionally, in order to minimize exposure of a subject to X-rays, an X-ray diagnostic apparatus includes an X-ray aperture capable of narrowing an irradiation field of X-rays to a region of interest. An operator can adjust a size of the irradiation field to correspond to the region of interest by adjusting the arrangement of the four X-ray aperture blades of the X-ray aperture via a console device. In the X-ray aperture, a size of an opening formed by the four X-ray aperture blades can be changed in a left-right direction and an up-down direction while a center position of the opening is fixed. Therefore, the operation of the X-ray aperture is performed by an adjustment lever that changes the size of the opening in the left-right direction and the up-down direction. Furthermore, the size of the opening can be switched to a predetermined size by one-touch operation using a button. Furthermore, in recent years, not only the size of the opening is changed in a state where the center position of the opening is fixed, but also the size of the opening can be changed while moving the center position of the opening by moving four X-ray aperture blades independently in the left-right direction and the up-down direction. Therefore, an operation device of the X-ray aperture includes not only a lever that changes the size of the opening but also a lever that changes the center position of the opening, that is, the position of the irradiation field.

However, in the conventional X-ray diagnostic apparatus, in a case where a visual field is adjusted to the region of interest, it is necessary to use a plurality of separate operation devices, and the operation is complicated. Furthermore, in the conventional X-ray diagnostic apparatus, it is difficult to intuitively grasp the size and position of the current irradiation field with respect to an entire X-ray detector. In particular, in the case that a captured image is displayed on a monitor while the visual field is enlarged, it is difficult to intuitively grasp the size and position of the current irradiation field with respect to the entire X-ray detector. In addition, in the conventional X-ray diagnostic apparatus, it is difficult for the operator to intuitively grasp what kind of operation should be performed in order to adjust the X-ray aperture blades to a desired arrangement.

Therefore, in the conventional X-ray diagnostic apparatus, it is difficult to improve the operability of the X-ray aperture.

US2023000449A1 relates to an X-ray diagnosis apparatus which includes an X-ray limiter having four diaphragm blades; and a console on which four physical operating units that correspond to the four diaphragm blades are placed at four positions. When viewed from the side of the operator of the console, the four operating units are placed on the far side, the near side, the left side, and the right side. The far-side operating unit, the near-side operating unit, the left-side operating unit, and the right-side operating unit correspond to the upper diaphragm blade, the lower diaphragm blade, the left-side diaphragm blade, and the right-side diaphragm blade, respectively, with reference to an X-ray image displayed in a display. An operation of moving the far-side operating unit in the far-side direction results in the movement of the upper diaphragm blade in the upward direction of the X-ray image displayed in the display, and an operation of moving the far-side operating unit in the near-side direction results in the movement of the upper diaphragm blade in the downward direction of the X-ray image displayed in the display. An operation of moving the near-side operating unit in the far-side direction results in the movement of the lower diaphragm blade in the upward direction of the X-ray image displayed in the display, and an operation of moving the near-side operating unit in the near-side direction results in the movement of the lower diaphragm blade in the downward direction of the X-ray image displayed in the display. An operation of moving the left-side operating unit in the leftward direction results in the movement of the left-side diaphragm blade in the leftward direction of the X-ray image displayed in the display, and an operation of moving the left-side operating unit in the rightward direction results in the movement of the left-side diaphragm blade in the rightward direction of the X-ray image displayed in the display. An operation of moving the right-side operating unit in the leftward direction results in the movement of the right-side diaphragm blade in the leftward direction of the X-ray image displayed in the display, and an operation of moving the right-side operating unit in the rightward direction results in the movement of the right-side diaphragm blade in the rightward direction of the X-ray image displayed in the display.

### SUMMARY OF INVENTION

In a first aspect, an X-ray diagnostic apparatus is provided as recited in claim 1.

The two operation sections disposed on at least one of the back side and the front side of the display section may respectively correspond to an X-ray aperture blade on a left side of an X-ray image displayed by the X-ray diagnostic apparatus and an X-ray aperture blade on a right side of the X-ray image, and
the two operation sections disposed on at least one of the left side and the right side of the display section may respectively correspond to an upper X-ray aperture blade and a lower X-ray aperture blade of the X-ray image displayed by the X-ray diagnostic apparatus.

A size of the first irradiation range may be greater than or equal to a size of the second irradiation range.

The size of the first irradiation range may be a size when the X-ray aperture is fully opened.

The two operation sections disposed on at least one of the back side and the front side of the display section may be movable to a left side and a right side as viewed from the operator, and
the two operation sections disposed on at least one of the left side and the right side of the display section may be movable to a back side and a front side as viewed from the operator.

The operation sections may be disposed, two each, on rails disposed in parallel to one side of the first irradiation range indicated by the display section and to another side not facing the one side.

The X-ray diagnostic apparatus may further include an imparting section that is configured to impart a click feeling to an operator through the operation assembly when a size of an irradiation field reaches a predetermined size.

The imparting section may comprise a contact member that comes into contact with the operation assembly when the size of the irradiation field reaches the predetermined size.

The predetermined size may include a plurality of sizes.

The processing circuitry may be configured to be able to control positions of the X-ray aperture such that a center of the second irradiation range is different from a center of the first irradiation range, according to an operation on the operation section.

The X-ray diagnostic apparatus may further include a designation section that is configured to designate a predetermined size of an irradiation field, wherein
the processing circuitry may be further configured to control positions of the X-ray aperture according to the designation of the size of the irradiation field by the designation section, and to cause the X-ray aperture to form the second irradiation range corresponding to the irradiation field of the size designated by the designation section, wherein
the processing circuitry may be further configured to move the operation assembly to a position corresponding to the second irradiation range in conjunction with formation of the second irradiation range.

The display section may be configured to indicate the first irradiation range by a physical frame.

The display section may be configured to indicate the first irradiation range by an image.

A second display section that is configured to indicate the second irradiation range formed by the X-ray apertures may be further provided.

The second display section may include a rod-shaped body extending toward the display section from each of two of the operation sections disposed on at least one of the back side and the front side of the display section and two of the operation sections disposed on at least one of the left side and the right side of the display section, and may indicate the second irradiation range by a region surrounded by the rod-shaped body.

The display section may be configured to indicate the second irradiation range by an image.

Each of the operation sections is a slider, and
the two operation sections disposed on at least one of the one side and the other side may include knobs having a positional relationship in which the knobs are linearly arranged in a movement direction of the two operation sections.

A movement direction of the X-ray apertures may be inclined with respect to a longitudinal direction and a lateral direction of a bed on which the subject is placed.

A movement direction of the X-ray apertures may be inclined with respect to a longitudinal direction and a lateral direction of a bed on which the subject is placed, and
each side of the first irradiation range indicated by the display section may be inclined with respect to a direction from a back side to a front side of the display section and a direction from a left side to a right side as viewed from the operator.

In a second aspect, a method of operating an irradiation field is provided as recited in claim 12.

### BRIEF DESCRIPTION OF THE DRAWINGS

FIG. 1 is a diagram illustrating an example of a configuration of an X-ray diagnostic apparatus according to a first embodiment.
FIG. 2 is a block diagram illustrating an example of a configuration of the X-ray diagnostic apparatus according to the first embodiment.
FIG. 3 is a plan view illustrating an X-ray aperture in the X-ray diagnostic apparatus according to the first embodiment.
FIG. 4 is a perspective view illustrating an X-ray tube, an X-ray aperture, and an X-ray detector in the X-ray diagnostic apparatus according to the first embodiment.
FIG. 5 is a plan view illustrating an operation section and a display section in a remote operation console according to the first embodiment.
FIG. 6 is a flowchart illustrating an operation example of the X-ray diagnostic apparatus according to the first embodiment.
FIG. 7 is a plan view illustrating an operation section and a display section in a remote operation console according to a first modification of the first embodiment.
FIG. 8 is a plan view illustrating an operation section and a display section in a remote operation console according to a second modification of the first embodiment.
FIG. 9 is a block diagram illustrating an example of a configuration of an X-ray diagnostic apparatus according to a second embodiment.
FIG. 10 is a plan view illustrating a visual field size changeover switch in a remote operation console according to the second embodiment.
FIG. 11 is a plan view illustrating an operation section, a display section, and an operation section movement mechanism in the remote operation console according to the second embodiment.
FIG. 12 is a flowchart illustrating an operation example of the X-ray diagnostic apparatus according to the second embodiment.
FIG. 13 is a plan view illustrating an operation example of the X-ray diagnostic apparatus according to the second embodiment.
FIG. 14 is a plan view illustrating an operation section, a display section, and an imparting section in a remote operation console according to a third embodiment.
FIG. 15 is a plan view illustrating a first operation example of the remote operation console according to the third embodiment.
FIG. 16 is a plan view illustrating a second operation example of the remote operation console according to the third embodiment.
FIG. 17 is a perspective view illustrating an operation section in a remote operation console according to a fourth embodiment.
FIG. 18 is a view of the operation section in the remote operation console according to the fourth embodiment as viewed from a movement direction of the operation section.
FIG. 19 is a view of an operation section in a remote operation console according to a first modification of the fourth embodiment as viewed from a movement direction of the operation section.
FIG. 20 is a perspective view illustrating an operation section in a remote operation console according to a second modification of the fourth embodiment.
FIG. 21 is a plan view illustrating an X-ray aperture and an operation section in an X-ray diagnostic apparatus according to a fifth embodiment.
FIG. 22 is a plan view illustrating an X-ray aperture and an operation section in an X-ray diagnostic apparatus according to a modification of the fifth embodiment.

### DETAILED DESCRIPTION

Hereinafter, embodiments of an X-ray diagnostic apparatus will be described with reference to the drawings. Note that in the following description, an X-ray diagnostic apparatus having an over-tube type configuration in which an X-ray tube is located above a subject will be described as an example of the X-ray diagnostic apparatus, but the X-ray diagnostic apparatus may be configured as an under-tube type in which the X-ray tube is located below the subject. Furthermore, the X-ray diagnostic apparatus may be applied to a single-plane X-ray diagnostic apparatus including one C-arm, a biplane X-ray diagnostic apparatus including two C-arms, an X-ray television apparatus, an X-ray CT apparatus, and the like. Furthermore, in the following description, constituent elements having substantially the same functions and configurations are denoted by the same reference signs, and redundant description will be made only when necessary.

### (First embodiment)

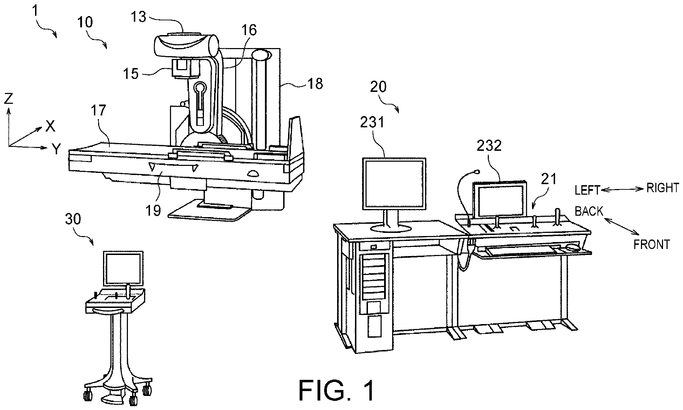

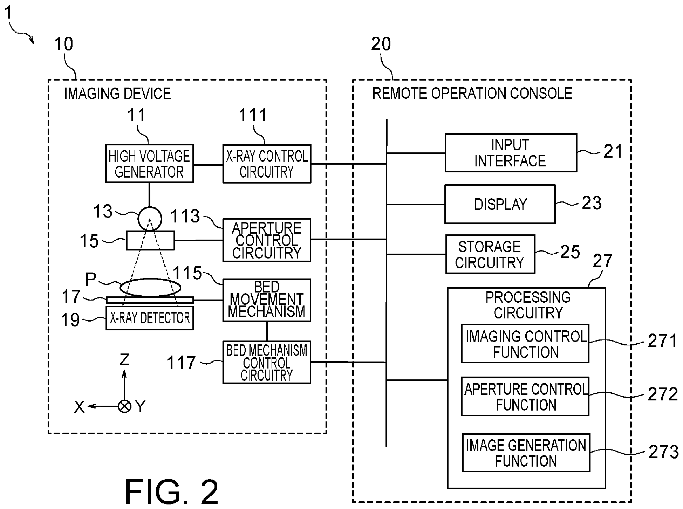

FIG. 1 is a diagram illustrating an example of a configuration of an X-ray diagnostic apparatus 1 according to a first embodiment. As illustrated in FIGS. 1 and 2, the X-ray diagnostic apparatus 1 according to the first embodiment includes an imaging device 10, a remote operation console 20, and a proximity operation console 30. The remote operation console 20 and the proximity operation console 30 are examples of a console device. The imaging device 10 is a device that images a subject using X-rays. The remote operation console 20 is, for example, a device for an operator to control an operation of the imaging device 10 in an operation room separate from an examination room where the imaging device 10 is located. The proximity operation console 30 is, for example, a device for the operator to control the operation of the imaging device 10 in the examination room. The imaging device 10, the remote operation console 20, and the proximity operation console 30 are communicably connected.

FIG. 2 is a block diagram illustrating an example of a configuration of the X-ray diagnostic apparatus 1 according to the first embodiment. Note that in FIG. 2, illustration of the proximity operation console 30 is omitted. As illustrated in FIGS. 1 and 2, the imaging device 10 includes a high-voltage generator 11, an X-ray tube 13, an X-ray aperture 15, a holding device 16, a bed 17, an X-ray detector 19, X-ray control circuitry 111, aperture control circuitry 113, a bed movement mechanism 115, and bed mechanism control circuitry 117. The remote operation console 20 includes an input interface 21, a display 23, storage circuitry 25, and processing circuitry 27. For easy understanding, in the following description, a direction along a longitudinal direction of the bed 17 is defined as a Y-axis direction, a direction along a lateral direction is defined as an X-axis direction, and a direction orthogonal to the X-axis direction and the Y-axis direction is defined as a Z-axis direction.

The high-voltage generator 11 is a device that supplies a high voltage and a filament current to the X-ray tube 13. The high-voltage generator 11 includes electric circuitry such as a transformer and a rectifier, and a high-voltage generation device. The high voltage generation device has a function of generating a high voltage to be applied to the X-ray tube 13 and a filament current to be supplied to the X-ray tube 13. The X-ray control circuitry 111 controls the supply of the high voltage and the filament current by the high voltage generation device. The high-voltage generation device may be a transformer type or an inverter type. Note that the high-voltage generator 11 may be provided in the holding device 16.

The X-ray tube 13 irradiates a subject P with an X-ray. The X-ray tube 13 is a vacuum tube that generates X-rays by irradiating a positive electrode (target) with thermoelectrons from a negative electrode (filament) by application of a high voltage and supply of a filament current from the high-voltage generator 11. In the X-ray tube 13, X-rays are generated by thermoelectrons colliding with a target. The X-ray tube 13 is, for example, a rotating anode type X-ray tube that generates an X-ray by irradiating a rotating anode with thermoelectrons. The type of the X-ray tube 13 is not limited to the rotating anode type, and any type can be applied.

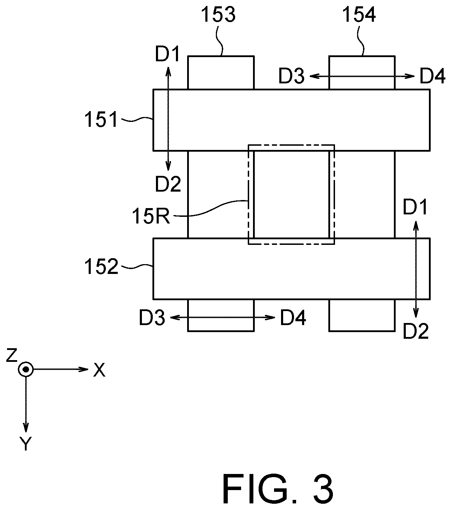

The X-ray aperture 15 narrows down the X-ray emitted from the X-ray tube 13. The X-ray aperture 15 is provided on a front surface of an X-ray emission window in the X-ray tube 13. FIG. 3 is a plan view illustrating the X-ray aperture 15 in the X-ray diagnostic apparatus 1 according to the first embodiment. As illustrated in FIG. 3, the X-ray aperture 15 includes four X-ray aperture blades 151, 152, 153, and 154 including a first X-ray aperture blade 151, a second X-ray aperture blade 152, a third X-ray aperture blade 153, and a fourth X-ray aperture blade 154.

The first X-ray aperture blade 151 is, for example, an X-ray aperture blade corresponding to an upper side of an X-ray image displayed by the X-ray diagnostic apparatus 1. That is, the first X-ray aperture blade 151 is an X-ray aperture blade that defines an upper end of the X-ray image displayed on the display 23. The second X-ray aperture blade 152 is, for example, an X-ray aperture blade corresponding to a lower side of the X-ray image. That is, the second X-ray aperture blade 152 is an X-ray aperture blade that defines a lower end of the X-ray image displayed on the display 23. The third X-ray aperture blade 153 is, for example, an X-ray aperture blade corresponding to a left side of the X-ray image. That is, the third X-ray aperture blade 153 is an X-ray aperture blade that defines a left end of the X-ray image displayed on the display 23. The fourth X-ray aperture blade 154 is, for example, an X-ray aperture blade corresponding to a right side of the X-ray image. That is, the fourth X-ray aperture blade 154 is an X-ray aperture blade that defines a right end of the X-ray image displayed on the display 23. The X-ray aperture blades 151, 152, 153, and 154 are made of, for example, a metal plate such as lead.

The relationship between the X-ray image displayed on the display 23 and the X-ray aperture blades is an example, and in a case where the X-ray image displayed on the display is vertically and horizontally inverted, the correspondence with the X-ray aperture blades changes.

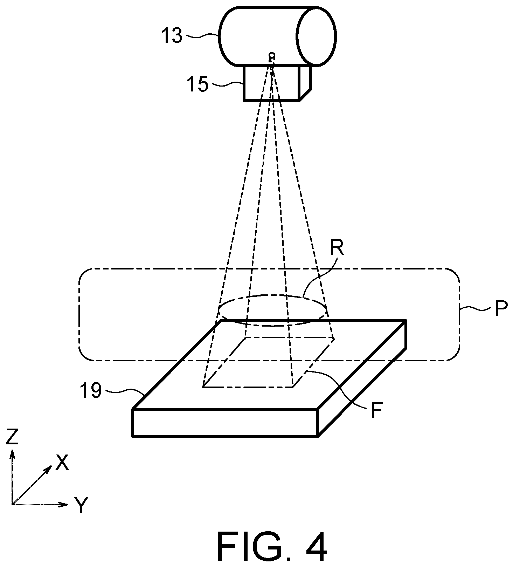

The X-ray aperture blades 151, 152, 153, and 154 are driven by the aperture control circuitry 113 according to a region of interest R of the subject P illustrated in FIG. 4 input by the operator via the input interface 21. Furthermore, the X-ray aperture blades 151, 152, 153, and 154 are driven by the aperture control circuitry 113 in accordance with an operation of operation assembly which comprises a plurality of operation sections 211, 212, 213, and 214 (described later) included in the input interface 21. The X-ray aperture blades 151, 152, 153, and 154 are driven by the aperture control circuitry 113 to slide, thereby limiting an irradiation range 15R of the X-ray emitted from the X-ray tube 13 to the subject P.

In the example illustrated in FIG. 3, the first X-ray aperture blade 151 and the second X-ray aperture blade 152 are slidable independently in a direction D1 along the Y-axis and a direction D2 opposite to the direction D1. Furthermore, the third X-ray aperture blade 153 and the fourth X-ray aperture blade 154 are slidable independently in a D3 direction orthogonal to the D1 direction along the X-axis and in a D4 direction opposite to the D3 direction. Since the X-ray aperture blades 151, 152, 153, and 154 are slidable independently, the size and position of irradiation range 15R formed by an opening surrounded by the X-ray aperture blades 151, 152, 153, and 154 can be arbitrarily adjusted. In other words, the X-ray aperture blades 151, 152, 153, and 154 can arbitrarily adjust the size and range of a region where the X-ray is shielded. As a result, the X-ray aperture 15 narrows down the X-ray generated by the X-ray tube 13 so that the region of interest R of the subject P is irradiated with the X-ray.

The X-ray detector 19 detects the X-ray generated by the X-ray tube 13 and transmitted through the subject P. The X-ray detector 19 is, for example, an X-ray flat panel detector (Hereinafter referred to as FPD). The FPD includes, for example, a plurality of semiconductor detection elements. Each of the semiconductor detection elements includes a direct conversion type in which an X-ray is directly converted into an electric signal and an indirect conversion type in which an X-ray is converted into light by a phosphor and the light is converted into an electric signal. Any format may be used for the FPD. Electrical signals generated by the plurality of semiconductor detection elements in association with incidence of X-rays are output to an analog to digital converter (Hereinafter referred to as A/D converter) not illustrated. The A/D converter converts the electrical signals into digital data. The A/D converter outputs the digital data to the processing circuitry 27. Note that an image intensifier may be used as the X-ray detector 19.

The holding device 16 holds the X-ray tube 13 and the X-ray aperture 15. The holding device 16 is movably supported by a stand 18 by a movement mechanism of the holding device 16 (not illustrated). The movement mechanism of the holding device 16 rotates the holding device 16 about, for example, a rotation axis along the X-axis under the control of the processing circuitry 27. Furthermore, the movement mechanism of the holding device 16 moves the holding device 16 along an upper surface of the bed 17, for example, under the control of the processing circuitry 27. The movement mechanism of the holding device 16 includes, for example, a drive source such as a motor provided on the stand 18, and a driving force transmission member that transmits a driving force of the drive source to the holding device 16.

The bed 17 is a device for placing the subject P. The bed 17 is moved by the bed movement mechanism 115 under the control of the bed mechanism control circuitry 117. The bed movement mechanism 115 adjusts the reclining of the bed 17, for example, by rotating the bed 17 about a rotation axis along the X-axis direction. The bed movement mechanism 115 includes, for example, a drive source such as a motor provided in the stand 18, and a driving force transmission member that transmits the driving force of the drive source to the bed 17.

The input interface 21 receives various instructions and information input operations from the operator. Specifically, the input interface 21 converts an input operation received from the operator into an electric signal and outputs the electric signal to the processing circuitry 27. For example, the input interface 21 is realized by a trackball, a switch button, a mouse, a keyboard, a touch pad that performs an input operation by touching an operation surface, a touch screen in which a display screen and the touch pad are integrated, non-contact input circuitry using an optical sensor, sound input circuitry, and the like. Note that the input interface 21 is not limited to one including physical operation components such as a mouse and a keyboard. For example, electric signal processing circuitry that receives an electric signal corresponding to an input operation from an external input device provided separately from the apparatus and outputs the electric signal to control circuitry is also included in the example of the input interface 21.

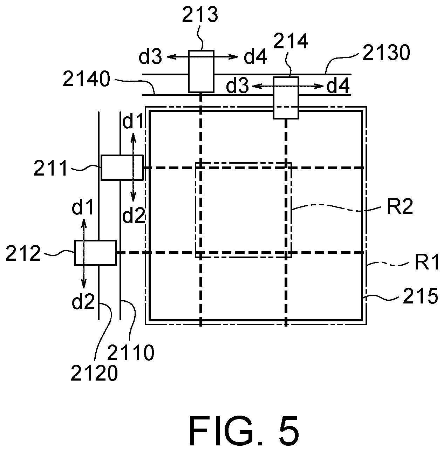

FIG. 5 is a plan view illustrating operation sections 211, 212, 213, and 214 and a display section 215 in the remote operation console 20 according to the first embodiment. As illustrated in FIG. 5, the input interface 21 includes the operation assembly comprising the four operation sections 211, 212, 213, and 214 including a first operation section 211, a second operation section 212, a third operation section 213, and a fourth operation section 214, and the display section 215. Each of the operation sections 211, 212, 213, and 214 includes, for example, a slider that outputs an electric signal indicating an input operation by linear motion. Each of the operation sections 211, 212, 213, and 214 may be a knob of a slider. Positions of the input operation using the operation sections 211, 212, 213, and 214 including the sliders may be calculated by the processing circuitry 27 using a sensor such as an encoder.

The operation sections 211, 212, 213, and 214 designate a position of each of the four X-ray aperture blades 151, 152, 153, and 154 of the X-ray aperture 15. Two operation sections 213 and 214 of the operation sections 211, 212, 213, and 214 are disposed on at least one of a back side and a front side of the display section 215 as viewed from the operator of the remote operation console 20. Two operation sections 211 and 212 of the operation sections 211, 212, 213, and 214 are disposed on at least one of a left side and a right side of the display section 215 as viewed from the operator. In FIG. 1, arrows indicate directions corresponding to the front side, the back side, the left side, and the right side of the display section 215 as viewed from the operator of the remote operation console 20. In the example illustrated in FIG. 5, the operation sections 211, 212, 213, and 214 are disposed two on the back side of the display section 215 as viewed from the operator, and two on the left side of the display section 215 as viewed from the operator. More specifically, in the example illustrated in FIG. 5, the first operation section 211 and the second operation section 212 are disposed on the left side of the display section 215 as viewed from the operator. The second operation section 212 is disposed on front side of the first operation section 211 as viewed from the operator. Furthermore, the third operation section 213 and the fourth operation section 214 are disposed on the back side of the display section 215 as viewed from the operator. The fourth operation section 214 is disposed to the right side of the third operation section 213 as viewed from the operator.

The first operation section 211 disposed on the left side of the display section 215 as viewed from the operator corresponds to the first X-ray aperture blade 151 on the upper side of the X-ray image displayed by the X-ray diagnostic apparatus 1. The second operation section 212 disposed on the left side of the display section 215 and in front of the first operation section 211 as viewed from the operator corresponds to the second X-ray aperture blade 152 on the lower side of the X-ray image. The third operation section 213 disposed on the back side of the display section 215 as viewed from the operator corresponds to the third X-ray aperture blade 153 on the left side of the X-ray image. The fourth operation section 214 disposed on the back side of the display section 215 and on the right side of the third operation section 213 as viewed from the operator corresponds to the fourth X-ray aperture blade 154 on the right side of the X-ray image.

The first operation section 211 and the second operation section 212 are movable toward the back and front as viewed from the operator. The third operation section 213 and the fourth operation section 214 are movable toward the left and right when viewed from the operator. The operation sections 211, 212, 213, and 214 are independently movable in each movement direction.

The display section 215 indicates a first irradiation range formed by the X-ray aperture 15. The first irradiation range is, for example, the irradiation range 15R formed when the X-ray aperture 15 is fully opened. In the example illustrated in FIG. 5, the display section 215 has a rectangular outline, and the first irradiation range is indicated by a region R1 surrounded by the outline. The display section 215 includes, for example, a physical frame, and indicates the first irradiation range by an outer shape of the frame. The display section 215 is not limited to including the physical frame, and for example, may electronically display a region indicating the first irradiation range on a display or a panel. That is, the display section 215 may indicate the first irradiation range as an image. Hereinafter, the region R1 indicating the first irradiation range in the display section 215 is also referred to as a first indication region R1. Note that the display section 215 has a configuration separate from a display section that displays a fluoroscopic image (that is, an X-ray captured image) such as a fluoroscopic monitor 232 described later. That is, the display section 215 is configured to indicate the first irradiation range (that is, first indication region R1) without displaying the fluoroscopic image.

The operation sections 211, 212, 213, and 214 are respectively disposed on rails 2110, 2120, 2130, and 2140 disposed in parallel with one side of the first irradiation range (that is, first indication region R1) indicated by the display section 215. The operation sections 211, 212, 213, and 214 are slidably disposed along the rails 2110, 2120, 2130, and 2140.

Specifically, the operation sections 211, 212, 213, and 214 are disposed, two each, on the rails 2110 and 2120 disposed in parallel to one side of the first indication region R1 and on the rails 2130 and 2140 disposed in parallel to another side not facing the one side.

More specifically, the first operation section 211 is disposed on the first rail 2110 disposed in parallel to the left and right sides as viewed from the operator in the first indication region R1. In the example illustrated in FIG. 5, the first rail 2110 is disposed on the left side of the display section 215 as viewed from the operator. The first operation section 211 is movable toward the back (that is, a d1 direction in FIG. 5) and the front (that is, in a d2 direction in FIG. 5,) as viewed from the operator along the first rail 2110. The second operation section 212 is disposed on the second rail 2120 disposed in parallel to the left and right sides as viewed from the operator in the first indication region R1. In the example illustrated in FIG. 5, the second rail 2120 is disposed along the first rail 2110 on the left side of the display section 215 as viewed from the operator. The second operation section 212 is movable toward the back (direction d1 in FIG. 5) and the front (direction d2 in FIG. 5) as viewed from the operator along the second rail 2120. The third operation section 213 is disposed on the third rail 2130 disposed in parallel to the back side and the front side as viewed from the operator in the first indication region R1. The third operation section 213 is movable toward the left (that is, in a d3 direction in FIG. 5,) and the right (that is, in a d4 direction in FIG. 5,) as viewed from the operator along the third rail 2130. The fourth operation section 214 is disposed on the fourth rail 2140 disposed in parallel to the back side and the front side as viewed from the operator in the first indication region R1. The fourth operation section 214 is movable toward the left (d3 direction in FIG. 5) and the right (d4 direction in FIG. 5) as viewed from the operator along the fourth rail 2140.

A movement range of the first operation section 211 may be restricted so as not to move to the front side of the second operation section 212 as viewed from the operator by hitting the second operation section 212 from the d2 direction. That is, a movement range of the second operation section 212 may be restricted so as not to move to the back side of the first operation section 211 as viewed from the operator by hitting the first operation section 211 from the d1 direction. Furthermore, the movement ranges of the first operation section 211 and the second operation section 212 may be restricted by lengths of the first rail 2110 and the second rail 2120 so as not to move the back side and the front side of the display section 215.

A movement range of the third operation section 213 may be restricted so as not to move to the right side of the fourth operation section 214 as viewed from the operator by hitting the fourth operation section 214 from the d4 direction. That is, the movement range of the fourth operation section 214 may be restricted so as not to move to the left side of the third operation section 213 as viewed from the operator by hitting the third operation section 213 from the d3 direction. Furthermore, the moving ranges of the third operation section 213 and the fourth operation section 214 may be restricted by lengths of the third rail 2130 and the fourth rail 2140 so as not to move in the d3 direction and the d4 direction with respect to the display section 215.

The display 23 illustrated in FIG. 2 is a device that displays various types of information. For example, the display 23 converts image data transmitted from the processing circuitry 27 into an electric signal for display and outputs the electric signal. The display 23 is realized by a liquid crystal monitor, a cathode ray tube (CRT) monitor, a touch panel, and the like. In the example illustrated in FIG. 1, the display 23 includes a system monitor 231 and a fluoroscopic monitor 232. For example, the system monitor 231 displays a captured image (still image), a fluoroscopic image (moving image), and the like as an X-ray image. The fluoroscopic monitor 232 displays, for example, a fluoroscopic image.

The storage circuitry 25 is a non-transitory storage device that stores various types of information, and is, for example, a hard disk drive (HDD), an optical disk, a solid state drive (SSD), an integrated circuitry storage device, or the like. The storage circuitry 25 stores, for example, a control program for controlling the X-ray diagnostic apparatus 1 and various data used for executing the control program. The storage circuitry 25 may be a drive device that reads and writes various types of information from and to a portable storage medium such as a compact disc (CD), a digital versatile disc (DVD), and a flash memory, a semiconductor memory element such as a random access memory (RAM), or the like, in addition to the HDD, the SSD, and the like.

The processing circuitry 27 is circuitry that controls the entire operation of the X-ray diagnostic apparatus 1 according to the electric signal of the input operation input from the input interface 21. For example, the processing circuitry 27 includes an imaging control function 271, an aperture control function 272, and an image generation function 273. The aperture control function 272 is an example of a controller.

Here, for example, each processing function executed by the imaging control function 271, the aperture control function 272, and the image generation function 273, which are constituent elements of the processing circuitry 27 illustrated in FIG. 2, is recorded in the storage circuitry 25 in the form of a program executable by a computer. The processing circuitry 27 is, for example, a processor. The processor constituting the processing circuitry 27 reads each program from the storage circuitry 25 and executes the program to implement a function corresponding to each read program. In other words, the processing circuitry 27 in a state of reading each program has each function illustrated in the processing circuitry 27 of FIG. 2.

Note that, in FIG. 2, a case where each processing function of the imaging control function 271, the aperture control function 272, and the image generation function 273 is realized by single processing circuitry 27 has been illustrated, but the embodiment is not limited thereto. For example, the processing circuitry 27 may be configured by combining a plurality of independent processors, and each processor may implement each processing function by executing each program. Furthermore, each processing function of the processing circuitry 27 may be implemented by being appropriately distributed or integrated into a single or a plurality of processing circuitry.

The imaging control function 271 controls an imaging operation of the subject P by the imaging device 10 on the basis of, for example, an input operation received from the operator via the input interface 21. The imaging control function 271 controls the imaging operation of the subject P by controlling the X-ray control circuitry 111, the aperture control circuitry 113, the bed mechanism control circuitry 117, the movement mechanism of the holding device 16, and the like. More specifically, the imaging control function 271 reads the control program stored in the storage circuitry 25, develops the control program on a memory in the processing circuitry 27, and controls each section of the X-ray diagnostic apparatus 1 according to the developed control program.

The aperture control function 272 controls the positions of the corresponding X-ray aperture blades 151, 152, 153, and 154 according to the operations on the operation assembly 211, 212, 213, and 214, and causes the X-ray aperture 15 to form a second irradiation range. A size of the second irradiation range is less than or equal to a size of the first irradiation range. The aperture control function 272 can control the position of the X-ray aperture 15 such that a center of the second irradiation range is different from a center of the first irradiation range according to the operations on the operation sections 211, 212, 213, and 214.

The image generation function 273 generates image data based on the output from the X-ray detector 19. The image data is data of a medical image including a fluoroscopic image and a captured image related to the subject P. The imaging control function 271 displays the generated image data on the display 23.

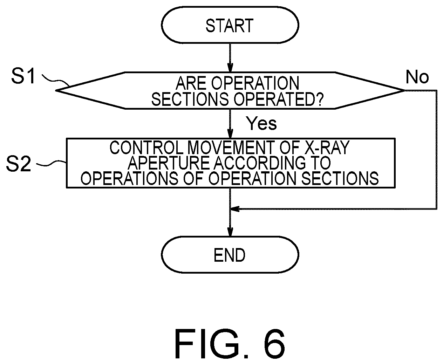

Next, an operation example of the X-ray diagnostic apparatus 1 according to the first embodiment configured as described above will be described. FIG. 6 is a flowchart illustrating an operation example of the X-ray diagnostic apparatus 1 according to the first embodiment. Note that the flowchart of FIG. 6 is repeated as necessary. First, as illustrated in FIG. 6, the aperture control function 272 determines whether or not the operator has operated the operation sections 211, 212, 213, and 214 (step S1).

In a case where the operation sections 211, 212, 213, and 214 are operated (Step S1: Yes), the aperture control function 272 performs movement control of the X-ray aperture 15 according to the operations of the operation sections 211, 212, 213, and 214 (step S2).

Specifically, the aperture control function 272 controls the aperture control circuitry 113 so as to move the first X-ray aperture blade 151 in the direction D1 in FIG. 3 by a movement amount according to an operation amount of the first operation section 211 in a case where the first operation section 211 is operated toward the back side (in the direction d1 in FIG. 5) as viewed from the operator. Furthermore, in a case where the first operation section 211 is operated toward the front side (in the d2 direction in FIG. 5) as viewed from the operator, the aperture control function 272 controls the aperture control circuitry 113 so as to move the first X-ray aperture blade 151 in the D2 direction in FIG. 3 by a movement amount according to an operation amount of the first operation section 211.

Furthermore, in a case where the second operation section 212 is operated toward the back side (in the d1 direction) as viewed from the operator, the aperture control function 272 controls the aperture control circuitry 113 so as to move the second X-ray aperture blade 152 in the D1 direction in FIG. 3 by a movement amount according to an operation amount of the second operation section 212. Furthermore, in a case where the second operation section 212 is operated toward the front side (in the d2 direction) as viewed from the operator, the aperture control function 272 controls the aperture control circuitry 113 so as to move the second X-ray aperture blade 152 in the D2 direction in FIG. 3 by a movement amount according to an operation amount of the second operation section 212.

Furthermore, in a case where the third operation section 213 is operated toward the left side (in the d3 direction in FIG. 5) as viewed from the operator, the aperture control function 272 controls the aperture control circuitry 113 so as to move the third X-ray aperture blade 153 in the D3 direction in FIG. 3 by a movement amount according to an operation amount of the third operation section 213. Furthermore, in a case where the third operation section 213 is operated toward the right side (in the direction d4 in FIG. 5) as viewed from the operator, the aperture control function 272 controls the aperture control circuitry 113 so as to move the third X-ray aperture blade 153 in the direction D4 in FIG. 3 by a movement amount according to an operation amount of the third operation section 213.

Furthermore, in a case where the fourth operation section 214 is operated toward the left side (in the d3 direction) as viewed from the operator, the aperture control function 272 controls the aperture control circuitry 113 so as to move the fourth X-ray aperture blade 154 in D3 direction in FIG. 3 by a movement amount according to an operation amount of the fourth operation section 214. Furthermore, in a case where the fourth operation section 214 is operated toward the right side (d4 direction) as viewed from the operator, the aperture control function 272 controls the aperture control circuitry 113 so as to move the fourth X-ray aperture blade 154 in the D4 direction in FIG. 3 by a movement amount according to an operation amount of the fourth operation section 214.

Note that an operation amount of each of the operation sections 211, 212, 213, and 214 is detected using, for example, a sensor such as an encoder. Specifically, the aperture control function 272 calculates the operation amount of each of the operation sections 211, 212, 213, and 214 based on an electric signal according to the operation amount of each of the operation sections 211, 212, 213, and 214 output from the sensor, and controls a movement of each of the X-ray aperture blades 151, 152, 153, and 154 based on the calculated operation amount. Furthermore, for example, the aperture control function 272 may determine the movement amounts of the X-ray aperture blades 151, 152, 153, and 154 according to the operation amounts of the operation sections 211, 212, 213, and 214 based on a correspondence relationship between the operation amounts of the operation sections 211, 212, 213, and 214 and movement amounts of the X-ray aperture blades 151, 152, 153, and 154 stored in the storage circuitry 25.

When operating the operation sections 211, 212, 213, and 214, as illustrated in FIG. 5, the operator can grasp a rectangular region R2 according to the positions of the operation sections 211, 212, 213, and 214 in the first indication region R1 of the display section 215. The region R2 is a region surrounded by an imaginary line (broken line in FIG. 5) extending from each of the operation sections 211, 212, 213, and 214 in a direction orthogonal to a sliding direction. The region R2 is a region indicating the second irradiation range formed by the X-ray aperture 15. Hereinafter, the region R2 is also referred to as a second indication region R2. Since the operator can operate the operation sections 211, 212, 213, and 214 while grasping the second indication region R2, the operator can intuitively grasp the size and position of the corresponding second irradiation range.

Note that similarly to the remote operation console 20, the configuration of the proximity operation console 30 may include the input interface 21 including the operation sections 211, 212, 213, and 214, the display 23, the storage circuitry 25, and the processing circuitry 277 including the aperture control function 272.

As described above, in the X-ray diagnostic apparatus 1 according to the first embodiment, the operation sections 211, 212, 213, and 214 (i.e., the operation assembly 211, 212, 213, and 214) respectively designate the positions of the four X-ray aperture blades 151, 152, 153, and 154 of the X-ray aperture 15 that limits the irradiation range of the X-ray with which the subject P is irradiated from the X-ray tube 13. The first indication region R1 of the display section 215 indicates the first irradiation range formed by the X-ray aperture 15. The aperture control function 272 controls the positions of the corresponding X-ray aperture blades 151, 152, 153, and 154 according to the operations on the operation sections 211, 212, 213, and 214 (i.e., the operation assembly 211, 212, 213, and 214), and causes the X-ray aperture 15 to form a second irradiation range.

As a result, it is not necessary to selectively use a plurality of different types of operation devices in order to adjust the visual field to the region of interest R, so that the operation of the X-ray aperture 15 becomes simple. The simple operation of the X-ray aperture 15 makes it possible to quickly and appropriately perform a highly urgent procedure such as interventional radiology (IVR). Furthermore, it is possible to intuitively grasp the size and position of the current X-ray irradiation field F with respect to the entire X-ray detector 19. In addition, the operator can intuitively grasp what kind of operation should be performed to adjust the X-ray aperture blades 151, 152, 153, and 154 to a desired arrangement. Therefore, the operability of the X-ray aperture 15 can be improved.

Furthermore, in the first embodiment, two operation sections of the operation sections 211, 212, 213, and 214 are disposed on at least one of the back side and the front side of the display section 215 as viewed from the operator of the remote operation console 20. Further, other two operation sections of the operation sections 211, 212, 213, and 214 are disposed on at least one of the left side and the right side of the display section 215 as viewed from the operator.

Consequently, the operation sections 211, 212, 213, and 214 can be disposed so as to be matched with the positional relationship of the X-ray aperture blades 151, 152, 153, and 154, so that the intuitiveness of the operation of the X-ray aperture 15 can be improved.

Furthermore, in the first embodiment, the two operation sections 213 and 214 disposed on at least one of the back side and the front side of the display section 215 as viewed from the operator respectively correspond to the X-ray aperture blade 153 on the left side of the X-ray image displayed by the X-ray diagnostic apparatus 1 and the X-ray aperture blade 154 on the right side of the X-ray image. Furthermore, the two operation sections 211 and 212 disposed on at least one of the left side and right side of the display section 215 as viewed from the operator correspond to the X-ray aperture blade 151 on the upper side and the X-ray aperture blade 152 on the lower side of the X-ray image displayed by the X-ray diagnostic apparatus 1, respectively.

As a result, the operation sections 211, 212, 213, and 214 can be disposed to further match the positional relationship of the X-ray aperture blades 151, 152, 153, and 154, so that the intuitiveness of the operation of the X-ray aperture 15 can be further improved.

Furthermore, in the first embodiment, the size of the first irradiation range is greater than or equal to the size of the second irradiation range.

As a result, the display section 215 can indicate the irradiation range when the X-ray aperture 15 is fully opened, so that the operation of the X-ray aperture 15 by the operation sections 211, 212, 213, and 214 can be more intuitively performed.

Furthermore, in the first embodiment, the two operation sections 213 and 214 disposed on at least one of the back side and the front side of the display section 215 as viewed from the operator are movable toward the left and the right as viewed from the operator. Furthermore, the two operation sections 211 and 212 disposed on at least one of the left side and the right side of the display section 215 as viewed from the operator are movable toward the back and the front as viewed from the operator.

Consequently, the movement directions of the operation sections 211, 212, 213, and 214 can be matched with the movement directions of the X-ray aperture blades 151, 152, 153, and 154, so that the intuitiveness of the operation of the X-ray aperture 15 can further be improved.

Furthermore, in the first embodiment, each of the operation sections 211, 212, 213, and 214 is a slider.

Consequently, the X-ray aperture 15 can easily be operated by the blind touch.

Furthermore, in the first embodiment, the operation sections 211, 212, 213, and 214 are disposed on the rails 2110, 2120, 2130, and 2140 disposed in parallel to one side of the first irradiation range (that is, first indication region R1) indicated by the display section 215.

With this simple configuration, the operation sections 211, 212, 213, and 214 can be moved so as to be aligned with the movement directions of the X-ray aperture blades 151, 152, 153, and 154.

Furthermore, in the first embodiment, the operation sections 211, 212, 213, and 214 are disposed, two each, on the rails 2110 and 2120 disposed in parallel to one side of the first irradiation range (first indication region R1) indicated by the display section 215 and on the rails 2130 and 2140 disposed in parallel to another side not facing the one side.

As a result, the operator can intuitively grasp the size and position of the second irradiation region formed by the X-ray aperture 15 based on the second indication region R2 defined in the display section 215 according to the positions of the operation sections 211, 212, 213, and 214 on the rails 2110, 2120, 2130, and 2140, so that the intuitiveness of the operation of the X-ray aperture 15 can be further improved.

Furthermore, in the first embodiment, the aperture control function 272 can control the position of the X-ray aperture 15 such that the center of the second irradiation range is different from the center of the first irradiation range according to the operations on the operation sections 211, 212, 213, and 214.

As a result, not only the size of the irradiation range but also the center position can be changed, so that the degree of freedom in selecting the irradiation field can be improved.

Note that various modifications described below can be applied to the first embodiment.

### (First modification)

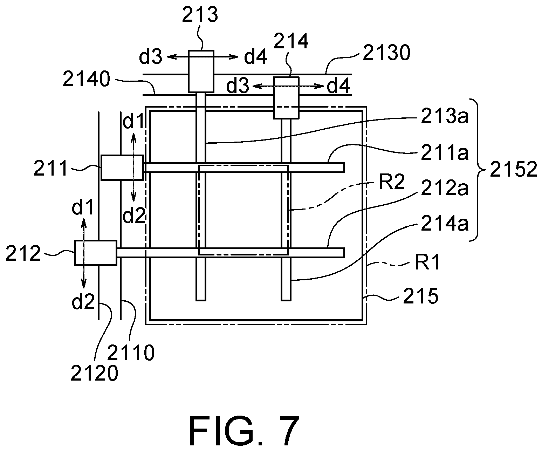

Next, a first modification of visualizing the second indication region R2 defined in the display section 215 according to the positions of the operation sections 211, 212, 213, and 214 will be described. FIG. 7 is a plan view illustrating the operation sections 211, 212, 213, and 214 and the display section 215 in the remote operation console 20 according to the first modification of the first embodiment.

The example in which the operator can grasp the second indication region R2 indicating the second irradiation range of the X-ray aperture 15 in the display section 215 according to the positions of the operation sections 211, 212, 213, and 214 has been described above. On the other hand, in the first modification, the operation sections 211, 212, 213, and 214 is configured to be able to visualize the second indication region R2.

Specifically, as illustrated in FIG. 7, in the first modification, the operation sections 211, 212, 213, and 214 include a second display section 2152 indicating a second irradiation range formed by the X-ray aperture 15 on the display section 215. The second display section 2152 includes four rod-shaped bodies 211a, 212a, 213a, and 214a extending from the operation sections 211, 212, 213, and 214 toward the display section 215 along directions orthogonal to the movement directions of the operation sections 211, 212, 213, and 214. Specifically, the second display section 2152 includes a first rod-shaped body 211a extending in the d4 direction from the first operation section 211, a second rod-shaped body 212a extending in the d4 direction from the second operation section 212, a third rod-shaped body 213a extending in the d2 direction from the third operation section 213, and a fourth rod-shaped body 214a extending in the d2 direction from the fourth operation section 214. The rod-shaped body 211a of the first operation section 211 intersects with the rod-shaped body 213a of the third operation section 213 and the rod-shaped body 214a of the fourth operation section 214. The rod-shaped body 212a of the second operation section 212 intersects with the rod-shaped body 213a of the third operation section 213 and the rod-shaped body 214a of the fourth operation section 214. The second display section 2152 can indicate the second irradiation range by a rectangular region (that is, second indication region R2) surrounded by the four rod-shaped bodies 211a, 212a, 213a, and 214a.

Note that in a case where the display section 215 is configured to display an image of the first indication region R1 indicating the first irradiation range on a display or a panel, the second display section 2152 may display an image of the second indication region R2 indicating the second irradiation range on the first indication region R1. In this case, the second display section 2152 may make a display mode (for example, display color, luminance, and the like) of the second indication region R2 different from that of the first indication region R1.

According to the first modification, since the second indication region R2 can be visualized, the intuitiveness of the operation of the X-ray aperture 15 can be further improved.

### (Second modification)

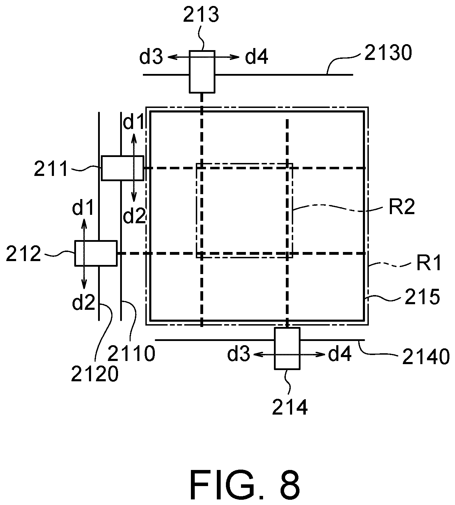

Next, a second modification in which the arrangement of the operation sections 211, 212, 213, and 214 is changed will be described. FIG. 8 is a plan view illustrating the operation sections 211, 212, 213, and 214 and the display section 215 in the remote operation console 20 according to the second modification of the first embodiment.

The example in which both the third operation section 213 and the fourth operation section 214 are disposed on the back side of the display section 215 as viewed from the operator has been described above. On the other hand, as illustrated in FIG. 8, the fourth operation section 214 may be disposed on the front side of the display section 215 as viewed from the operator. Alternatively, the third operation section 213 may also be disposed on the front side of the display section 215 as viewed from the operator. Alternatively, the third operation section 213 may be disposed on the front side of the display section 215 as viewed from the operator, and the fourth operation section 214 may be disposed on the back side of the display section 215 as viewed from the operator. Furthermore, at least one of the first operation section 211 and the second operation section 212 may be disposed on the right side of the display section 215 as viewed from the operator.

According to the second modification, the degree of freedom in design can be improved by changing the arrangement of the operation sections 211, 212, 213, and 214.

### (Second embodiment)

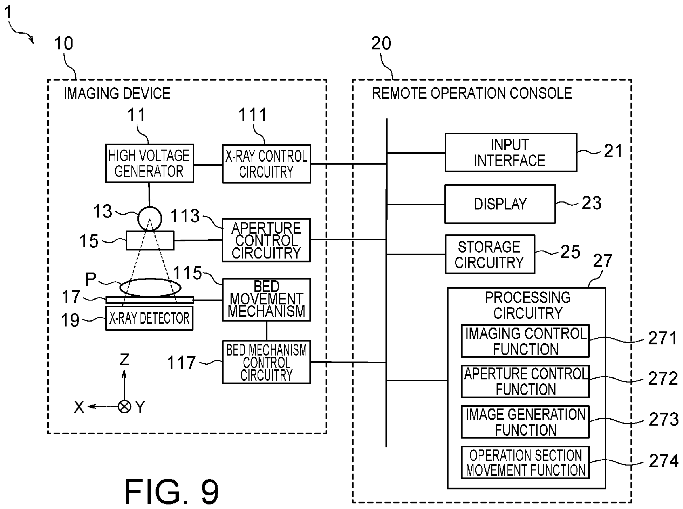

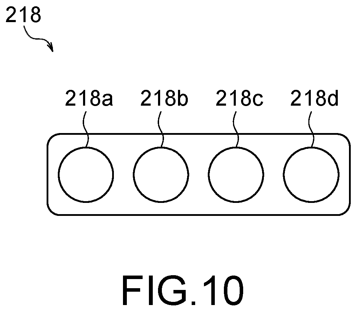

Next, a second embodiment in which the operation sections 211, 212, 213, and 214 are moved according to the set visual field size will be described focusing on a difference from the first embodiment. FIG. 9 is a block diagram illustrating an example of a configuration of the X-ray diagnostic apparatus 1 according to the second embodiment. FIG. 10 is a plan view illustrating a visual field size changeover switch 218 in the remote operation console 20 according to the second embodiment.

As illustrated in FIG. 9, in the second embodiment, the processing circuitry 27 of the remote operation console 20 further includes an operation section movement function 274 in addition to the configuration of the first embodiment. The operation section movement function 274 is an example of a moving section. Furthermore, as illustrated in FIG. 10, in the second embodiment, the input interface 21 of the remote operation console 20 further includes a visual field size changeover switch 218 in addition to the configuration of the first embodiment. The visual field size changeover switch 218 is an example of a designation section.

The visual field size changeover switch 218 is a switch that designates a predetermined size of the irradiation field. In the example illustrated in FIG. 10, the visual field size changeover switch 218 includes a first switch 218a, a second switch 218b, a third switch 218c, and a fourth switch 218d. The first switch 218a is a switch that designates a size of the maximum irradiation field. The second switch 218b is a switch that designates a size of the second largest irradiation field. The third switch 218c is a switch that designates a size of the third largest irradiation field. The fourth switch 218d is a switch that designates a size of the minimum irradiation field. The size of the irradiation field designated by the first switch 218a may be, for example, a fixed size. The size of the irradiation field designated by the second switch 218b, the third switch 218c, and the fourth switch 218d may be selectable from a plurality of options at the time of initial setting.

The aperture control function 272, which is an example of a second controller, controls the positions of the X-ray aperture blades 151, 152, 153, and 154 of the X-ray aperture 15 according to the designation of the size of the irradiation field by the visual field size changeover switch 218, and causes the X-ray aperture 15 to form the second irradiation range corresponding to the irradiation field having the size designated by the visual field size changeover switch 218. For example, when the operator presses one of the switches 218a, 218b, 218c, and 218d, an electric signal that designates the size of the irradiation field corresponding to the pressed switch 218a, 218b, 218c, or 218d is output to the aperture control function 272. The aperture control function 272 controls the aperture control circuitry 113 to cause the X-ray aperture 15 to form the second irradiation range corresponding to the irradiation field of the designated size according to the electrical signal. In a case where the visual field size changeover switch 218 is operated after the operations on the operation sections 211, 212, 213, and 214, the aperture control function 272 may perform control to move the X-ray aperture 15 so that the second irradiation range corresponding to the irradiation field of the size designated by the visual field size changeover switch 218 is formed without changing a center position of the second irradiation range formed by the operations on the operation sections 211, 212, 213, and 214.

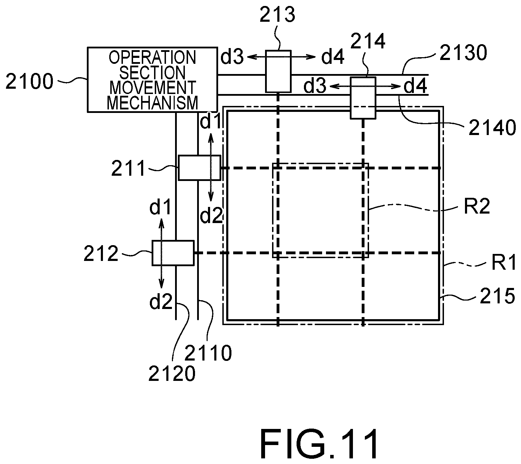

The operation section movement function 274 moves the operation sections 211, 212, 213, and 214 to positions corresponding to the second irradiation range in conjunction with the formation of the second irradiation range by the aperture control function 272. For example, the operation section movement function 274 electrically controls an operation section movement mechanism 2100 illustrated in FIG. 11 to move the operation sections 211, 212, 213, and 214 to positions corresponding to the second irradiation range in conjunction with the formation of the second irradiation range by the aperture control function 272. The operation section movement mechanism 2100 is a mechanism that moves the operation sections 211, 212, 213, and 214 under the control of the operation section movement function 274. The operation section movement mechanism 2100 can include, for example, drive sources such as motors corresponding to the operation sections 211, 212, 213, and 214, respectively, and a driving force transmission member such as a gear that transmits a driving force of each drive source to the corresponding operation sections 211, 212, 213, and 214. Note that the operation section movement mechanism 2100 may cut off a transmission path of the driving force between the drive sources and the operation sections 211, 212, 213, and 214 except when the visual field size changeover switch 218 is operated so that the manual operation of the operation sections 211, 212, 213, and 214 is not hindered when the drive source is stopped.

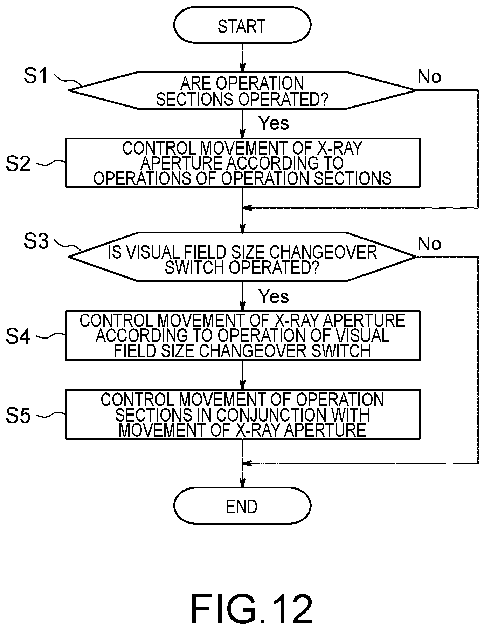

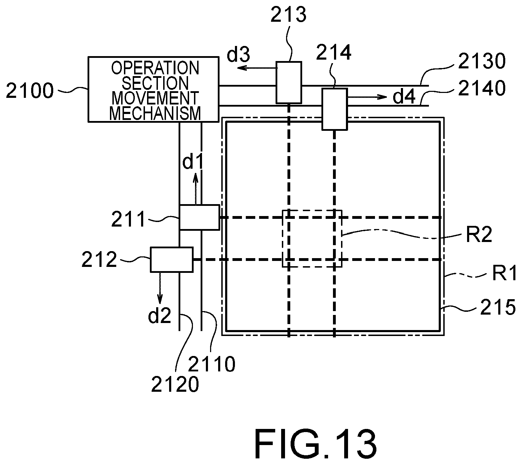

FIG. 12 is a flowchart illustrating an operation example of the X-ray diagnostic apparatus 1 according to the second embodiment. FIG. 13 is a plan view illustrating an operation example of the X-ray diagnostic apparatus 1 according to the second embodiment.

As illustrated in FIG. 12, in the second embodiment, after the movement control (step S2) of the X-ray aperture 15 according to the operations of the operation sections 211, 212, 213, and 214 is performed, the aperture control function 272 determines whether or not the visual field size changeover switch 218 is operated (step S3). Furthermore, in order to cope with the case where only the visual field size changeover switch 218 is operated, in the second embodiment, the aperture control function 272 also determines whether or not the visual field size changeover switch 218 is operated (step S3) in a case where the movement control (step S2) of the X-ray aperture 15 according to the operations of the operation sections 211, 212, 213, and 214 is not performed (step S1: No).

In a case where the visual field size changeover switch 218 is operated (step S3: Yes), the aperture control function 272 performs movement control of the X-ray aperture 15 according to the operation of the visual field size changeover switch 218 (step S4). For example, the aperture control function 272 performs control to move the X-ray aperture 15 so that the second irradiation range corresponding to the irradiation field of the size designated by the visual field size changeover switch 218 is formed without changing the center position of the second irradiation range formed by the operations on the operation sections 211, 212, 213, and 214.

After the movement control of the X-ray aperture 15 according to the operation of the visual field size changeover switch 218 is performed, the operation section movement function 274 performs the movement control of the operation sections 211, 212, 213, and 214 in conjunction with the movement of the X-ray aperture 15 (step S5).

For example, as illustrated in FIG. 13, in a case where the visual field size changeover switch 218 designates an irradiation field having a size larger than the irradiation field formed by the operations of the operation sections 211, 212, 213, and 214, the X-ray aperture blades 151, 152, 153, and 154 are moved such that the second irradiation range having a size larger than the current second irradiation range is formed. Accordingly, as illustrated in FIG. 13, the first operation section 211 is moved in the d1 direction. Furthermore, the second operation section 212 is moved in the d2 direction. Furthermore, the third operation section 213 is moved in the d3 direction. Furthermore, the fourth operation section 214 is moved in the d4 direction.

As described above, in the X-ray diagnostic apparatus 1 according to the second embodiment, the visual field size changeover switch 218 designates the predetermined size of the irradiation field. The aperture control function 272 controls the position of the X-ray aperture 15 according to the designation of the size of the irradiation field by the visual field size changeover switch 218, and causes the X-ray aperture 15 to form the second irradiation range corresponding to the irradiation field of the size designated by the visual field size changeover switch 218. The operation section movement function 274 moves the operation sections 211, 212, 213, and 214 (i.e., the operation assembly 211, 212, 213, and 214) to positions corresponding to the second irradiation range in conjunction with the formation of the second irradiation range by the aperture control function 272.

Thus, in a case where the size of the irradiation field is changed by the operation of the visual field size changeover switch 218, the positional relationship of the operation sections 211, 212, 213, and 214 can be matched with the changed irradiation field, so that the intuitiveness of the operation of the X-ray aperture 15 can be further improved.

### (Third embodiment)

Next, a third embodiment in which a click feeling is imparted to the operator through the operation sections 211, 212, 213, and 214 when the size of the irradiation field becomes a predetermined size will be described focusing on a difference from the first embodiment.

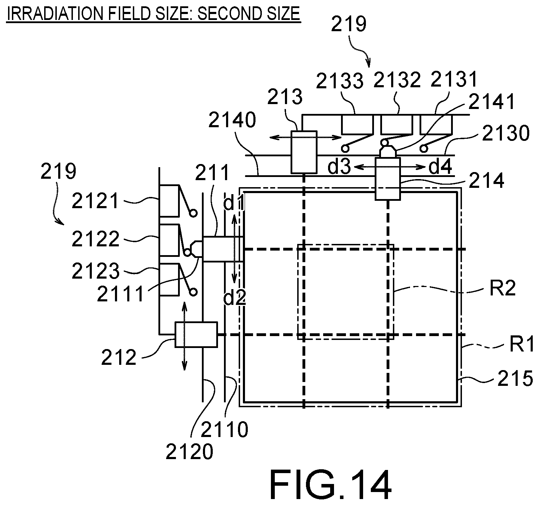

FIG. 14 is a plan view illustrating the operation sections 211, 212, 213, and 214 and the display section 215 in the remote operation console 20 according to the third embodiment. As illustrated in FIG. 14, the remote operation console 20 according to the third embodiment further includes an imparting section 219 that imparts a click feeling to the operator through the operation assembly 211, 212, 213, and 214 when the size of the irradiation field reaches a predetermined size, in addition to the configuration of the first embodiment. The predetermined size of the irradiation field includes a plurality of sizes.

More specifically, in the example illustrated in FIG. 14, the imparting section 219 includes a first contact member 2111 provided in the first operation section 211, first contact member groups 2121, 2122, and 2123 provided in the second operation section 212, a second contact member 2141 provided in the fourth operation section 214, and second contact member groups 2131, 2132, and 2133 provided in the third operation section 213.

The first contact member 2111 is a protruding member provided on the first operation section 211 so as to protrude from the first operation section 211 in the d3 direction opposite to the display section 215. The first contact member groups 2121, 2122, and 2123 are member groups that come into contact with the first operation section 211 of the operation assembly 211, 212, 213, and 214 via the first contact member 2111 when the size of the irradiation field becomes a plurality of predetermined sizes. The first contact member groups 2121, 2122, and 2123 are provided in the second operation section 212 so as to be located in the d3 direction with respect to the first contact member 2111. The first contact member groups 2121, 2122, and 2123 come into contact with the first contact member 2111 when a distance between the first operation section 211 and the second operation section 212 in a direction along the first rail 2110 and the second rail 2120 becomes a distance corresponding to each of the plurality of predetermined sizes of the irradiation field. The first contact member groups 2121, 2122, and 2123 include a first-group first contact member 2121, a first-group second contact member 2122, and a first-group third contact member 2123, which are disposed at intervals in the direction along the first rail 2110 and the second rail 2120. The first-group first contact member 2121 is disposed at a position farthest from the second operation section 212 among the first contact member groups 2121, 2122, and 2123. The first-group third contact member 2123 is disposed at a position closest to the second operation section 212 among the first contact member groups 2121, 2122, and 2123.

When the distance between the first operation section 211 and the second operation section 212 becomes a distance corresponding to a predetermined first size of the irradiation field, the first-group first contact member 2121 comes into contact with the first contact member 2111, and imparts a click feeling to the operator through the first operation section 211 by an impact force generated by the contact. When the distance between the first operation section 211 and the second operation section 212 becomes a distance corresponding to a predetermined second size of the irradiation field, the first-group second contact member 2122 comes into contact with the first contact member 2111, and imparts a click feeling to the operator through the first operation section 211 by an impact force generated by the contact. The second size is smaller than the first size. When the distance between the first operation section 211 and the second operation section 212 becomes a distance corresponding to a predetermined third size of the irradiation field, the first-group third contact member 2123 comes into contact with the first contact member 2111, and imparts a click feeling to the operator through the first operation section 211 by an impact force generated by the contact. The third size is smaller than the second size. The first contact member groups 2121, 2122, and 2123 are configured to be elastically deformable by contact with the first contact member 2111 so as not to hinder the movement of the first operation section 211 when coming into contact with the first contact member 2111. The first contact member groups 2121, 2122, and 2123 are, for example, at least partially made of an elastic body such as a spring.

The second contact member 2141 is a protruding member provided on the fourth operation section 214 so as to protrude from the fourth operation section 214 in the d1 direction opposite to the display section 215. The second contact member groups 2131, 2132, and 2133 are member groups that come into contact with the fourth operation section 214 of the operation assembly 211, 212, 213, and 214 via the second contact member 2141 when the size of the irradiation field becomes a plurality of predetermined sizes. The second contact member groups 2131, 2132, and 2133 are provided in the third operation section 213 so as to be located in the d1 direction with respect to the second contact member 2141. The second contact member groups 2131, 2132, and 2133 come into contact with the second contact member 2141 when a distance between the third operation section 213 and the fourth operation section 214 in a direction along the third rail 2130 and the fourth rail 2140 becomes a distance corresponding to each of a plurality of predetermined sizes of the irradiation field. The second contact member groups 2131, 2132, and 2133 include a second-group first contact member 2131, a second-group second contact member 2132, and a second-group third contact member 2133, which are disposed at intervals in the direction along the third rail 2130 and the fourth rail 2140. The second-group first contact member 2131 is disposed at a position farthest from the third operation section 213 among the second contact member groups 2131, 2132, and 2133. The second-group third contact member 2133 is disposed at a position closest to the third operation section 213 among the second contact member groups 2131, 2132, and 2133.

When the distance between the third operation section 213 and the fourth operation section 214 becomes a distance corresponding to the first size of the irradiation field, the second-group first contact member 2131 comes into contact with the second contact member 2141, and imparts a click feeling to the operator through the fourth operation section 214 by an impact force generated by the contact. When the distance between the third operation section 213 and the fourth operation section 214 becomes a distance corresponding to the second size of the irradiation field, the second-group second contact member 2132 comes into contact with the second contact member 2141, and imparts a click feeling to the operator through the fourth operation section 214 by an impact force generated by the contact. When the distance between the third operation section 213 and the fourth operation section 214 becomes a distance corresponding to the third size of the irradiation field, the second-group third contact member 2133 comes into contact with the second contact member 2141, and imparts a click feeling to the operator through the fourth operation section 214 by an impact force generated by the contact. The second contact member groups 2131, 2132, and 2133 are configured to be elastically deformable by contact with the second contact member 2141 so as not to hinder the movement of the fourth operation section 214 when coming into contact with the second contact member 2141. The second contact member groups 2131, 2132, and 2133 are, for example, at least partially made of an elastic body such as a spring.

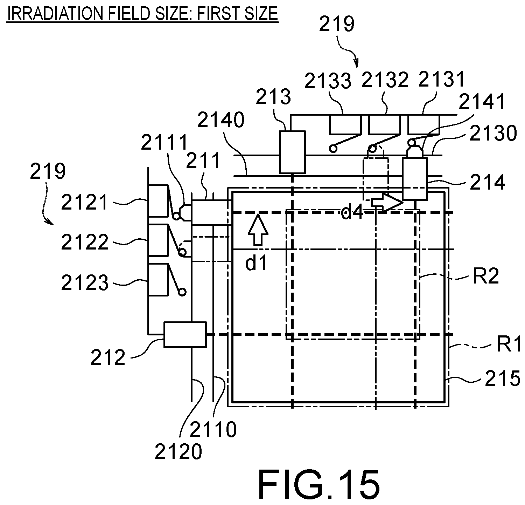

FIG. 15 is a plan view illustrating a first operation example of the remote operation console 20 according to the third embodiment. For example, it is assumed that the operator operates the first operation section 211 in the d1 direction and operates the fourth operation section 214 in the d4 direction from a state where the size of the irradiation field is the second size as illustrated in FIG. 14. By such operations of the first operation section 211 and the fourth operation section 214, the size of the irradiation field is enlarged from the second size. Then, as illustrated in FIG. 15, when the size of the irradiation field becomes the first size, the first-group first contact member 2121 comes into contact with the first contact member 2111, and a click feeling is imparted to the operator through the first operation section 211 by an impact force generated by the contact. Furthermore, when the size of the irradiation field becomes the first size, the second-group first contact member 2131 comes into contact with the second contact member 2141, and a click feeling is imparted to the operator through the fourth operation section 214 by an impact force generated by the contact. By imparting a click feeling through the first operation section 211 and the fourth operation section 214, the operator can easily grasp that the irradiation field has become the first size.

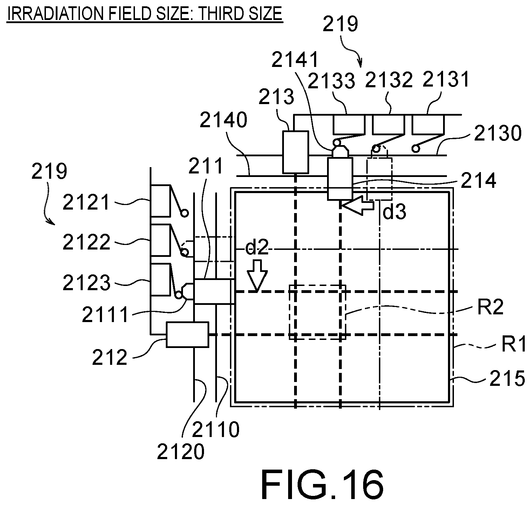

FIG. 16 is a plan view illustrating a second operation example of the remote operation console 20 according to the third embodiment. Furthermore, for example, it is assumed that the operator operates the first operation section 211 in the d2 direction and operates the fourth operation section 214 in the d3 direction from a state where the size of the irradiation field is the second size as illustrated in FIG. 14. By such operations of the first operation section 211 and the fourth operation section 214, the size of the irradiation field is reduced from the second size. Then, as illustrated in FIG. 16, when the size of the irradiation field becomes the third size, the first-group third contact member 2123 comes into contact with the first contact member 2111, and a click feeling is imparted to the operator through the first operation section 211 by an impact force generated by the contact. Furthermore, when the size of the irradiation field becomes the third size, the second-group third contact member 2133 comes into contact with the second contact member 2141, and a click feeling is imparted to the operator through the fourth operation section 214 by an impact force generated by the contact. By imparting a click feeling through the first operation section 211 and the fourth operation section 214, the operator can easily grasp that the irradiation field has become the third size.

Heretofore, the example has been described in which the imparting section 219 imparts a click feeling to the operator by the impact force generated by the contact between the first contact member 2111 and the first contact member groups 2121, 2122, and 2123 and the impact force generated by the contact between the second contact member 2141 and the second contact member groups 2131, 2132, and 2133. The present invention is not limited to imparting the click feeling to the operator by the impact forces. For example, the imparting section 219 may generate vibration according to the electrical contact between the first contact member 2111 and the first contact member groups 2121, 2122, and 2123 and the electrical contact between the second contact member 2141 and the second contact member groups 2131, 2132, and 2133, and impart a click feeling by the vibration to the operator.

For example, at least one of the first contact member 2111 and the second contact member 2141 may include a piezoelectric element. Furthermore, the imparting section 219 may include first electric circuitry that makes the first contact member 2111, the first-group first contact member 2121, the second contact member 2141, the second-group first contact member 2131, and a power supply conductive when the first contact member 2111 comes into contact with the first-group first contact member 2121 and the second contact member 2141 comes into contact with the second-group first contact member 2131. Furthermore, the imparting section 219 may include second electric circuitry that makes the first contact member 2111, the first-group second contact member 2122, the second contact member 2141, the second-group second contact member 2132, and a power supply conductive when the first contact member 2111 comes into contact with the first-group second contact member 2122 and the second contact member 2141 comes into contact with the second-group second contact member 2132. Furthermore, the imparting section 219 may include third electric circuitry that makes the first contact member 2111, the first-group third contact member 2123, the second contact member 2141, the second-group third contact member 2133, and a power supply conductive when the first contact member 2111 comes into contact with the first-group third contact member 2123 and the second contact member 2141 comes into contact with the second-group third contact member 2133. According to such a configuration, when the size of the irradiation field becomes each of the first size, the second size, and the third size, a click feeling can be imparted to the operator by the vibration generated by the piezoelectric element.

As described above, in the X-ray diagnostic apparatus 1 according to the third embodiment, when the size of the irradiation field becomes the predetermined size, the imparting section 219 impart the click feeling to the operator through the operation sections 211 and 214 of the operation assembly 211, 212, 213, and 214.

As a result, the operator can easily grasp that the size of the irradiation field becomes the predetermined size, so that the intuitiveness of the operation of the X-ray aperture 15 can be further improved.

### (Fourth embodiment)

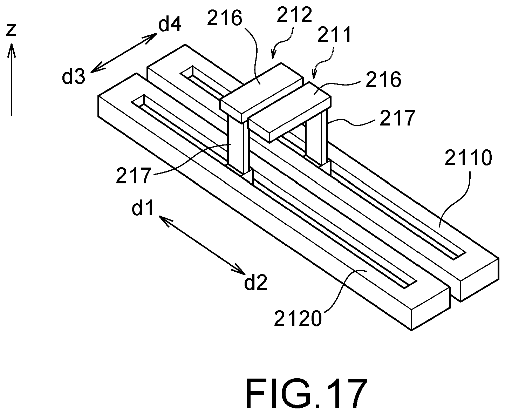

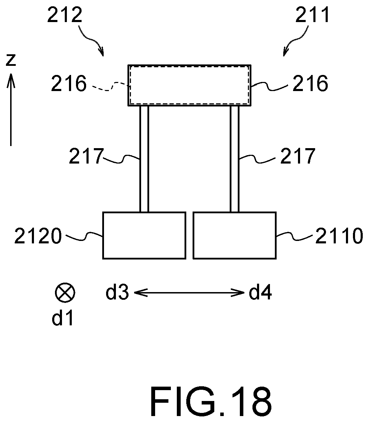

Next, a fourth embodiment in which the knobs of the operation section disposed on each of the two adjacent rails have a positional relationship in which the knobs are linearly arranged in the movement direction of the operation section will be described focusing on a difference from the above-described embodiments. FIG. 17 is a perspective view illustrating the operation sections 211 and 212 in the remote operation console 20 according to the fourth embodiment. FIG. 18 is a view of the operation sections 211 and 212 in the remote operation console 20 according to the fourth embodiment as viewed from the movement direction of the operation sections 211 and 212.

In the example illustrated in FIGS. 17 and 18, the first operation section 211 and the second operation section 212 disposed on the left side (that is, in the d3 direction) of the display section 215 as viewed from the operator include knobs 216 and 216 having a positional relationship in which the knobs are linearly arranged in the movement directions d1 and d2 of the first operation section 211 and the second operation section 212. The knob 216 of the first operation section 211 and the knob 216 of the second operation section 212 may have a positional relationship in which they overlap each other in the movement directions d1 and d2 of the first operation section 211 and the second operation section 212. That is, as illustrated in FIG. 18, the positions and ranges of the knob 216 of the first operation section 211 and the knob 216 of the second operation section 212 in the direction from the left (d3) to the right (d4) and the up-down direction (that is, in the Z-axis direction) as viewed from the operator may be aligned with each other.

The knobs 216 of the operation sections 211 and 212 are connected to the corresponding rails 2110 and 2120 via connection portions 217. In the example illustrated in FIGS. 17 and 18, the connection portion 217 of the first operation section 211 linearly extends upward (that is, in the Z-axis direction) from the first rail 2110 to the knob 216 of the first operation section 211. Furthermore, the connection portion 217 of the second operation section 212 extends linearly upward from the second rail 2120 to the knob 216 of the second operation section 212. Note that the operation sections 211, 212, 213, and 214 illustrated in each of the drawings of the first to third embodiments may be the knob 216 similar to that of the fourth embodiment, and the knobs 216 may be connected to the rails 2110, 2120, 2130, and 2140 via the connection portions 217.

According to the fourth embodiment, the knob 216 of the first operation section 211 and the knob 216 of the second operation section 212 have a positional relationship in which they are linearly arranged in the movement directions d1 and d2 of the first operation section 211 and the second operation section 212.

Accordingly, aesthetics and operability of the first operation section 211 and the second operation section 212 can be improved.

### (First modification)

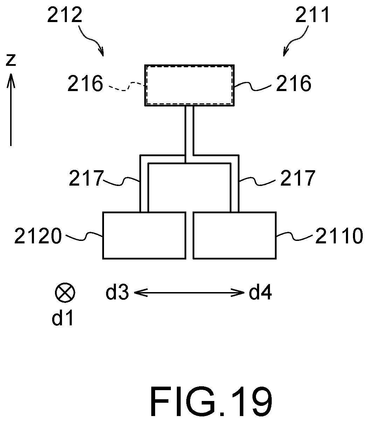

FIG. 19 is a view of the operation sections 211 and 212 in the remote operation console 20 according to a first modification of the fourth embodiment as viewed from the movement directions d1 and d2 of the operation sections 211 and 212. In FIGS. 17 and 18, the example in which the knobs 216 are connected to the rails 2110 and 2120 via the connection portions 217 linearly extending upward from the rails 2110 and 2120 has been described. On the other hand, in the example illustrated in FIG. 19, the connection portion 217 of the first operation section 211 extends upward from the first rail 2110 to the knob 216 while being bent to a left side d3 as viewed from the operator. Furthermore, the connection portion 217 of the second operation section 212 extends upward from the second rail 2120 to the knob 216 while being bent to a right side d4 as viewed from the operator.

According to the example illustrated in FIG. 19, by bending the connection portions 217, the knobs 216 of the operation sections 211 and 212 can be linearly arranged in the movement directions d1 and d2 without the need to increase the dimensions of the knobs 216 of the operation sections 211 and 212 in the directions d3 and d4 orthogonal to the movement directions d1 and d2.

### (Second modification)

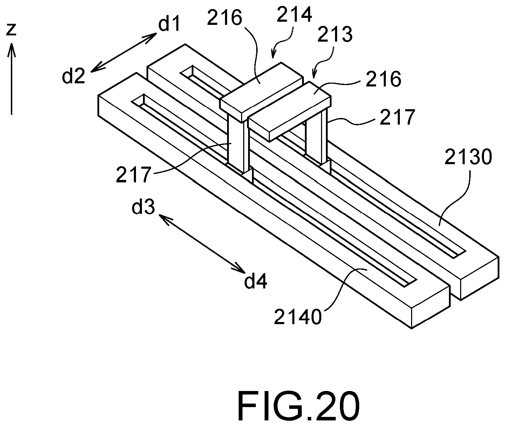

FIG. 20 is a perspective view illustrating the operation sections 213 and 214 in the remote operation console 20 according to a second modification of the fourth embodiment. The positional relationship between the knob 216 of the first operation section 211 and the knob 216 of the second operation section 212 described with reference to FIGS. 17 and 18 can also be applied to the third operation section 213 and the fourth operation section 214. Specifically, as illustrated in FIG. 20, the third operation section 213 and the fourth operation section 214 disposed on the back side of the display section 215 as viewed from the operator include the knobs 216 and 216 having a positional relationship in which the knobs are linearly arranged in the movement directions d3 and d4 of the third operation section 213 and the fourth operation section 214. The knob 216 of the third operation section 213 and the knob 216 of the fourth operation section 214 may have a positional relationship in which they overlap each other in the movement directions d3 and d4 of the third operation section 213 and the fourth operation section 214. That is, the knob 216 of the third operation section 213 and the knob 216 of the fourth operation section 214 may be aligned in position and range in a direction from the back side (d1) toward the front side (d2) and the up-down direction as viewed from the operator.

In the example illustrated in FIG. 20, the knobs 216 are connected to the rails 2130 and 2140 via the connection portions 217 linearly extending upward from the rails 2130 and 2140.

According to the example illustrated in FIG. 20, the knob 216 of the third operation section 213 and the knob 216 of the fourth operation section 214 have a positional relationship in which they are linearly arranged in the movement directions d3 and d4 of the third operation section 213 and the fourth operation section 214. Accordingly, aesthetics and operability of the third operation section 213 and the fourth operation section 214 can be improved. Note that the configuration of the operation sections 213 and 214 illustrated in FIG. 20 may be combined with the configuration of the operation sections 211 and 212 illustrated in FIGS. 17 and 18. Furthermore, the bent connection portion 217 illustrated in FIG. 19 may be applied to the operation sections 213 and 214 illustrated in FIG. 20.

### (Fifth embodiment)

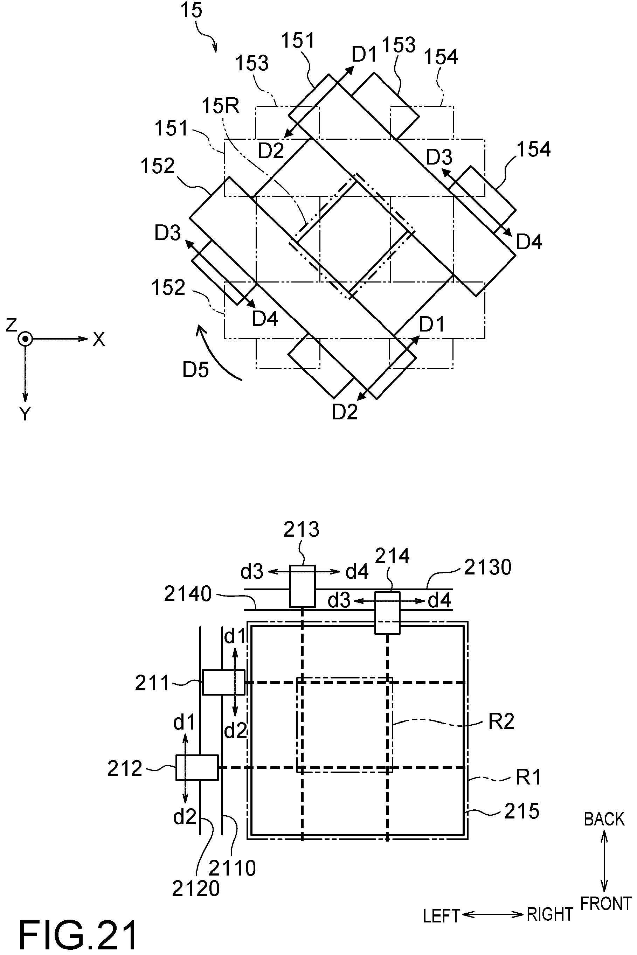

Next, a fifth embodiment in which the movement direction of the X-ray aperture 15 is inclined with respect to the longitudinal direction and the lateral direction of the bed 17 will be described focusing on a difference from the above-described embodiments. FIG. 21 is a plan view illustrating the X-ray aperture 15 and the operation sections 211, 212, 213, and 214 in the X-ray diagnostic apparatus 1 according to the fifth embodiment.

In FIG. 3, the example has been described in which the movement directions D1 and D2 of the first X-ray aperture blade 151 and the second X-ray aperture blade 152 are parallel to the longitudinal direction (that is, the Y-axis direction) of the bed 17, and the movement directions of the third X-ray aperture blade 153 and the fourth X-ray aperture blade 154 are parallel to the lateral direction (that is, the X-axis direction) of the bed 17. On the other hand, in the example illustrated in FIG. 21, the movement directions D1, D2, D3, and D4 of the X-ray aperture blades 151, 152, 153, and 154 are inclined with respect to the longitudinal direction and the lateral direction of the bed 17. More specifically, in the example illustrated in FIG. 21, the entire X-ray aperture 15 rotates about the Z-axis as indicated by an arrow D5 by driving the X-ray aperture 15 by the aperture control circuitry 113 according to the operation of the input interface 21. As the entire X-ray aperture 15 rotates, the movement directions D1, D2, D3, and D4 of the X-ray aperture blades 151, 152, 153, and 154 change so as to be inclined with respect to the longitudinal direction and the lateral direction of the bed 17.

Note that in FIG. 21, the X-ray aperture blades 151, 152, 153, and 154 before the entire X-ray aperture 15 is rotated are indicated by a two-dot chain line. A specific aspect of the input interface 21 for rotating the entire X-ray aperture 15 is not particularly limited. For example, an operation section for rotating the entire X-ray aperture 15 may be disposed in the vicinity of the operation sections 211, 212, 213, and 214. Alternatively, the entire display section 215 may be configured to be manually rotatable, and the entire X-ray aperture 15 may be configured to rotate by a rotation amount according to a rotation amount of the display section 215. Alternatively, the operation section for rotating the entire X-ray aperture 15 may be disposed away from the display section 215.

According to the fifth embodiment, since it is possible to reduce the work of selectively using a plurality of different types of operating devices in order to adjust the visual field to the region of interest R, it is possible to improve the operability of the X-ray aperture 15. Furthermore, by rotating the X-ray aperture 15, the degree of freedom in selecting a visual field can be improved.

### (Modification)

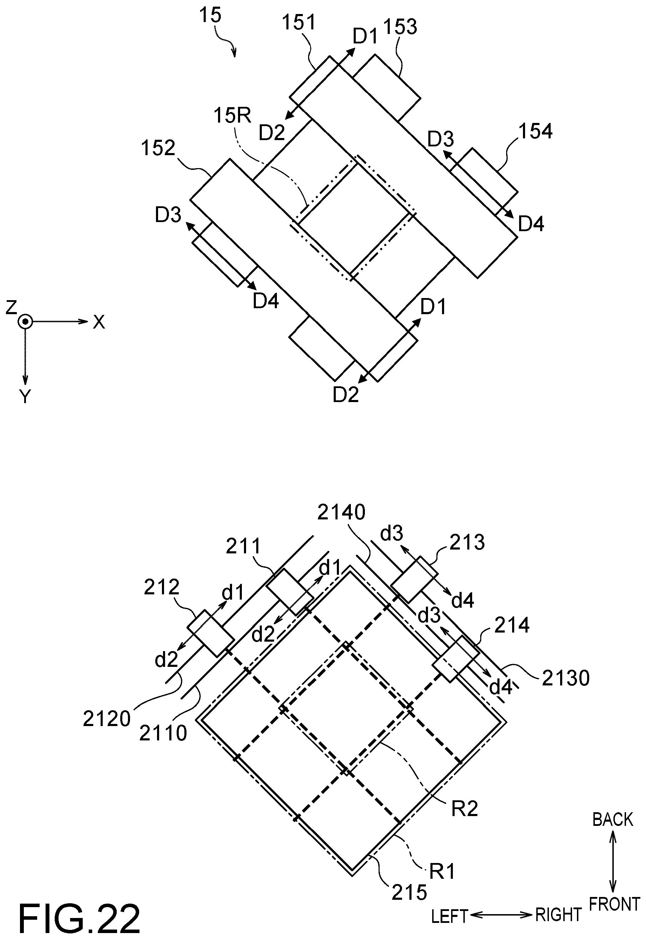

FIG. 22 is a plan view illustrating an X-ray aperture and an operation section in the X-ray diagnostic apparatus according to a modification of the fifth embodiment. In FIG. 21, the example in which the movement directions D1, D2, D3, and D4 of the X-ray aperture blades 151, 152, 153, and 154 change so as to be inclined with respect to the longitudinal direction and the lateral direction of the bed 17 by the rotation of the entire X-ray aperture 15 has been described. On the other hand, in the example illustrated in FIG. 22, the entire X-ray aperture 15 does not rotate, and the movement directions D1, D2, D3, and D4 of the X-ray aperture blades 151, 152, 153, and 154 are fixed in a state of being inclined with respect to the longitudinal direction and the lateral direction of the bed 17. Furthermore, each side of the first irradiation range R1 indicated by the display section 215 is inclined with respect to a direction from the back side to the front side and a direction from the left side to the right side of the display section 215 as viewed from the operator.

According to the example illustrated in FIG. 22, since the first irradiation range R1 and the movement directions of the operation sections 211, 212, 213, and 214 can be inclined in accordance with the inclinations of the movement directions D1, D2, D3, and D4 of the X-ray aperture blades 151, 152, 153, and 154, the X-ray aperture 15 can be operated more intuitively.

Note that the term "processor" used in the above description means, for example, a central processing unit (CPU), a graphics processing unit (GPU), or circuitry such as an application specific integrated circuit (ASIC) or a programmable logic device (for example, a simple programmable logic device (SPLD), a complex programmable logic device (CPLD), and a field programmable gate array (FPGA)). The processor realizes the function by reading and executing the program stored in the storage circuit. Note that instead of storing the program in the storage circuit, the program may be directly incorporated in the circuitry of the processor. In this case, the processor realizes the function by reading and executing a program incorporated in the circuit. Note that the processor is not limited to a case of being configured as a single processor circuit, and a plurality of independent circuits may be combined to be configured as one processor to realize the function. Moreover, the plurality of constituent elements in FIG. 2 may be integrated into one processor to implement the functions.

According to at least one embodiment described above, the operability of the X-ray aperture can be improved.

Although several embodiments have been described above, these embodiments have been presented only as examples, and are not intended to limit the scope of the invention. The novel devices and methods described herein can be implemented in a variety of other forms. Furthermore, various omissions, substitutions, and changes can be made to the forms of the apparatus and the method described in the present specification without departing from the scope of the invention as defined by the appended claims.

## Claims

1. An X-ray diagnostic apparatus (1) comprising:
an X-ray tube (13) configured to irradiate a subject with an X-ray;
an X-ray detector configured to detect an X-ray that has passed through the subject;
an X-ray aperture (15) comprising four X-ray aperture blades (151, 152, 153, 154) configured to limit an irradiation range of an X-ray with which the subject is irradiated from the X-ray tube (13); and
a console device (20),
wherein the console device (20) comprises
an operation assembly comprising a plurality of operation sections (211, 212, 213, 214) configured to designate a position of each of the four X-ray aperture blades (151, 152, 153, 154),
a display section (215) configured to indicate a first irradiation range formed by the X-ray aperture (13), and
processing circuitry (27) configured to control, according to an operation on the operation assembly (211, 212, 213, 214), a position of a corresponding X-ray aperture blade (151, 152, 153, 154) and cause the X-ray aperture (13) to form a second irradiation range,
**characterised in that**
the display section (215) is configured separately from a second display section (23) that displays an X-ray image,
each of the operation sections (211, 212, 213, 214) is a slider, and two of the operation sections (213, 214) of the plurality of operation sections are disposed on at least one of a back side and a front side of the display section (215), and two of the operation sections (211, 212) of the plurality of operation sections are disposed on at least one of a left side and a right side of the display section (215).

2. The X-ray diagnostic apparatus according to claim 1, wherein
the two operation sections (213, 214) disposed on at least one of the back side and the front side of the display section (215) respectively correspond to an X-ray aperture blade on a left side of an X-ray image displayed by the X-ray diagnostic apparatus and an X-ray aperture blade on a right side of the X-ray image, and
the two operation sections (211, 212) disposed on at least one of the left side and the right side of the display section (215) respectively correspond to an upper X-ray aperture blade and a lower X-ray aperture blade of the X-ray image displayed by the X-ray diagnostic apparatus.

3. The X-ray diagnostic apparatus according to any preceding claim, wherein a size of the first irradiation range (15R) is greater than or equal to a size of the second irradiation range.

4. The X-ray diagnostic apparatus according to claim 1, wherein
the two operation sections (213, 214) disposed on at least one of the back side and the front side of the display section (215) are movable to a left side and a right side, and
the two operation sections (211, 212) disposed on at least one of the left side and the right side of the display section are movable to a back side and a front side.

5. The X-ray diagnostic apparatus according to claim 4, wherein each of the operation sections (211, 212, 213, 214) is disposed on a rail (2110, 2120, 2130, 2140) disposed in parallel with one side of the first irradiation range indicated by the display section (215).

6. The X-ray diagnostic apparatus according to claim 1, further comprising an imparting section (219) that is configured to impart a click feeling to an operator through the operation assembly (211, 212, 213, 214) when a size of an irradiation field reaches a predetermined size.

7. The X-ray diagnostic apparatus according to claim 6, wherein the imparting section (219) comprises a contact member (2111) that comes into contact with the operation assembly (211, 212, 213, 214) when the size of the irradiation field reaches the predetermined size.

8. The X-ray diagnostic apparatus according to claim 6, wherein the predetermined size includes a plurality of sizes.

9. The X-ray diagnostic apparatus according to any one of claims 1 to 8, wherein the processing circuitry is configured to be able to control positions of the X-ray aperture (15) such that a center of the second irradiation range is different from a center of the first irradiation range (15R), according to an operation on the operation section.

10. The X-ray diagnostic apparatus according to claim 1, further comprising a designation section (218) that is configured to designate a predetermined size of an irradiation field, wherein
the processing circuitry (27) is further configured to control positions of the X-ray aperture (15) according to the designation of the size of the irradiation field by the designation section (218), and to cause the X-ray aperture (15) to form the second irradiation range corresponding to the irradiation field of the size designated by the designation section (218), wherein
the processing circuitry (27) is further configured to move the operation assembly (211, 212, 213, 214) to a position corresponding to the second irradiation range in conjunction with formation of the second irradiation range.

11. The X-ray diagnostic apparatus according to claim 5,
the two operation sections (211, 212, 213, 214) include knobs having a positional relationship in which the knobs overlap each other in a movement direction of the two operation sections.

12. A method of operating an irradiation field, the method comprising:
designating, by an operation assembly (211, 212, 213, 214), a position of each of four X-ray aperture blades (151, 152, 153, 154) included in an X-ray aperture (15) that limits an irradiation range of an X-ray with which a subject is irradiated from an X-ray tube (13);
indicating, by a display section (215), a first irradiation range (15R) formed by the X-ray aperture (15); and
controlling, according to an operation on the operation assembly (211, 212, 213, 214), a position of a corresponding X-ray aperture blade (151, 152, 153, 154) and causing the X-ray aperture (15) to form a second irradiation range,
**characterised in that**
the display section (215) is configured separately from a second display section (23) that displays an X-ray image,
each of the operation sections (211, 212, 213, 214) is a slider, and
two of the operation sections (213, 214) of the plurality of operation sections are disposed on at least one of a back side and a front side of the display section (215), and two of the operation sections (211, 212) of the plurality of operation sections are disposed on at least one of a left side and a right side of the display section (215).

## Patentansprüche

1. Röntgendiagnoseeinrichtung (1), umfassend:
eine Röntgenröhre (13), die dazu konfiguriert ist, ein Subjekt mit einem Röntgenstrahl zu bestrahlen;
einen Röntgendetektor, der dazu konfiguriert ist, einen Röntgenstrahl zu erfassen, der das Subjekt durchdrungen hat;
eine Röntgenblende (15), die vier Röntgenblendenlamellen (151, 152, 153, 154) umfasst, die dazu konfiguriert sind, einen Bestrahlungsbereich eines Röntgenstrahls zu begrenzen, mit dem das Subjekt von der Röntgenröhre (13) bestrahlt wird; und
eine Konsolenvorrichtung (20),
wobei die Konsolenvorrichtung (20) umfasst
eine Bedienanordnung, die eine Vielzahl von Bedienabschnitten (211, 212, 213, 214) umfasst, die dazu konfiguriert sind, eine Position jeder der vier Röntgenblendenlamellen (151, 152, 153, 154) zu bestimmen,
einen Anzeigeabschnitt (215), der dazu konfiguriert ist, einen ersten Bestrahlungsbereich anzugeben, der durch die Röntgenblende (13) gebildet wird, und
eine Verarbeitungsschaltung (27), die dazu konfiguriert ist, gemäß einer Bedienung der Bedienanordnung (211, 212, 213, 214) eine Position einer entsprechenden Röntgenblendenlamelle (151, 152, 153, 154) zu steuern und die Röntgenblendenlamelle (13) zu veranlassen, einen zweiten Bestrahlungsbereich zu bilden,
**dadurch gekennzeichnet, dass**
der Anzeigeabschnitt (215) getrennt von einem zweiten Anzeigeabschnitt (23) konfiguriert ist, der ein Röntgenbild anzeigt,
jeder der Bedienabschnitte (211, 212, 213, 214) ein Schieber ist, und
zwei der Bedienabschnitte (213, 214) der Vielzahl von Bedienabschnitten auf mindestens einer von einer Rückseite und einer Vorderseite des Anzeigeabschnitts (215) angeordnet sind und zwei der Bedienabschnitte (211, 212) der Vielzahl von Bedienabschnitten auf mindestens einer von einer linken und einer rechten Seite des Anzeigeabschnitts (215) angeordnet sind.

2. Röntgendiagnoseeinrichtung nach Anspruch 1, wobei
die zwei Bedienabschnitte (213, 214), die auf mindestens einer der Rückseite und der Vorderseite des Anzeigeabschnitts (215) angeordnet sind, jeweils einer Röntgenblendenlamelle auf der linken Seite eines von der Röntgendiagnoseeinrichtung angezeigten Röntgenbilds und einer Röntgenblendenlamelle auf einer rechten Seite des Röntgenbilds entsprechen, und
die zwei Bedienabschnitte (211, 212), die auf mindestens einer der linken und der rechten Seite des Anzeigeabschnitts (215) angeordnet sind, jeweils einer oberen Röntgenblendenlamelle und einer unteren Röntgenblendenlamelle des von der Röntgendiagnoseeinrichtung angezeigten Röntgenbilds entsprechen.

3. Röntgendiagnoseeinrichtung nach einem vorstehenden Anspruch, wobei eine Größe des ersten Bestrahlungsbereichs (15R) größer oder gleich einer Größe des zweiten Bestrahlungsbereichs ist.

4. Röntgendiagnoseeinrichtung nach Anspruch 1, wobei
die zwei Bedienabschnitte (213, 214), die auf mindestens einer der Rückseite und der Vorderseite des Anzeigeabschnitts (215) angeordnet sind, zu einer linken Seite und einer rechten Seite verschiebbar sind, und
die zwei Bedienabschnitte (211, 212), die auf mindestens einer der linken Seite und der rechten Seite des Anzeigeabschnitts angeordnet sind, zu einer Rückseite und einer Vorderseite verschiebbar sind.

5. Röntgendiagnoseeinrichtung nach Anspruch 4,
wobei jeder der Bedienabschnitte (211, 212, 213, 214) auf einer Schiene (2110, 2120, 2130, 2140) angeordnet ist, die parallel zu einer Seite des durch den Anzeigeabschnitt (215) angegebenen ersten Bestrahlungsbereichs angeordnet ist.

6. Röntgendiagnoseeinrichtung nach Anspruch 1, die weiter einen Vermittlungsabschnitt (219) umfasst, der dazu konfiguriert ist, einem Bediener über die Bedienanordnung (211, 212, 213, 214) ein Klickgefühl zu vermitteln, wenn eine Größe eines Bestrahlungsfelds eine vorbestimmte Größe erreicht.

7. Röntgendiagnoseeinrichtung nach Anspruch 6,
wobei der Vermittlungsabschnitt (219) ein Kontaktelement (2111) umfasst, das mit der Betriebsanordnung (211, 212, 213, 214) in Kontakt kommt, wenn die Größe des Bestrahlungsfelds die vorbestimmte Größe erreicht.

8. Röntgendiagnoseeinrichtung nach Anspruch 6,
wobei die vorbestimmte Größe eine Vielzahl von Größen beinhaltet.

9. Röntgendiagnoseeinrichtung nach einem der Ansprüche 1 bis 8,
wobei die Verarbeitungsschaltung dazu konfiguriert ist, Positionen der Röntgenblende (15) gemäß einer Bedienung des Bedienabschnitts so steuern zu können, dass sich ein Mittelpunkt des zweiten Bestrahlungsbereichs von einem Mittelpunkt des ersten Bestrahlungsbereichs (15R) unterscheidet.

10. Röntgendiagnoseeinrichtung nach Anspruch 1, die weiter einen Bereitstellungsabschnitt (218) umfasst, der dazu konfiguriert ist, eine vorbestimmte Größe eines Bestrahlungsfelds bereitzustellen, wobei
die Verarbeitungsschaltung (27) weiter dazu konfiguriert ist, Positionen der Röntgenblende (15) gemäß der Bereitstellung der Größe des Bestrahlungsfelds durch den Bereitstellungsabschnitt (218) zu steuern und die Röntgenblende (15) zu veranlassen, den zweiten Bestrahlungsbereich entsprechend dem Bestrahlungsfeld der durch den Bereitstellungsabschnitt (218) bereitgestellten Größe zu bilden, wobei
die Verarbeitungsschaltung (27) weiter dazu konfiguriert ist, die Bedienanordnung (211, 212, 213, 214) in Verbindung mit Bildung des zweiten Bestrahlungsbereichs in eine Position zu bewegen, die dem zweiten Bestrahlungsbereich entspricht.

11. Röntgendiagnoseeinrichtung nach Anspruch 5,
die zwei Bedienabschnitte (211, 212, 213, 214) Knöpfe beinhalten, die eine Positionsbeziehung aufweisen, in der die Knöpfe einander in einer Bewegungsrichtung der zwei Bedienabschnitte überlappen.

12. Verfahren zum Betreiben einer Bestrahlungsfelds, wobei das Verfahren Folgendes umfasst:
Bereitstellen, durch eine Bedienanordnung (211, 212, 213, 214), einer Position jeder der vier Röntgenblendenlamellen (151, 152, 153, 154), die in einer Röntgenblende (15) beinhaltet sind, die einen Bestrahlungsbereich eines Röntgenstrahls begrenzt, mit der ein Subjekt von einer Röntgenröhre (13) bestrahlt wird;
Angeben, durch einen Anzeigeabschnitt (215), eines ersten Bestrahlungsbereichs (15R), der von der Röntgenblende (15) gebildet wird; und
Steuern, gemäß einer Bedienung der Bedienanordnung (211, 212, 213, 214), einer Position einer entsprechenden Röntgenblendenlamelle (151, 152, 153, 154) und Veranlassen der Röntgenblende (15), einen zweiten Bestrahlungsbereich zu bilden,
**dadurch gekennzeichnet, dass**
der Anzeigeabschnitt (215) getrennt von einem zweiten Anzeigeabschnitt (23) konfiguriert ist, der ein Röntgenbild anzeigt,
jeder der Bedienabschnitte (211, 212, 213, 214) ein Schieber ist, und
zwei der Bedienabschnitte (213, 214) der Vielzahl von Bedienabschnitten auf mindestens einer von einer Rückseite und einer Vorderseite des Anzeigeabschnitts (215) angeordnet sind und zwei der Bedienabschnitte (211, 212) der Vielzahl von Bedienabschnitten auf mindestens einer von einer linken und einer rechten Seite des Anzeigeabschnitts (215) angeordnet sind.

## Revendications

1. Appareil de diagnostic à rayons X (1) comprenant :
un tube à rayons X (13) configuré pour irradier un sujet avec un rayon X ;
un détecteur de rayons X configuré pour détecter un rayon X qui a traversé le sujet ;
une ouverture à rayons X (15) comprenant quatre lames d'ouverture à rayons X (151, 152, 153, 154) configurées pour limiter une plage d'irradiation d'un rayon X avec lequel le sujet est irradié à partir du tube à rayons X (13) ; et
un dispositif de console (20),
dans lequel le dispositif de console (20) comprend
un ensemble d'opération comprenant une pluralité de sections d'opération (211, 212, 213, 214) configurées pour désigner une position de chacune des quatre lames d'ouverture à rayons X (151, 152, 153, 154),
une section d'affichage (215) configurée pour indiquer une première plage d'irradiation formée par l'ouverture à rayons X (13), et
un ensemble de circuits de traitement (27) configuré pour commander, selon une opération sur l'ensemble d'opération (211, 212, 213, 214), une position d'une lame d'ouverture à rayons X (151, 152, 153, 154) correspondante et amener l'ouverture à rayons X (13) à former une seconde plage d'irradiation,
**caractérisé en ce que**
la section d'affichage (215) est configurée séparément d'une seconde section d'affichage (23) qui affiche une image radiographique,
chacune des sections d'opération (211, 212, 213, 214) est un glisseur, et
deux des sections d'opération (213, 214) de la pluralité de sections d'opération sont disposées sur au moins un parmi un côté arrière et un côté avant de la section d'affichage (215), et deux des sections d'opération (211, 212) de la pluralité de sections d'opération sont disposées sur au moins un parmi un côté gauche et un côté droit de la section d'affichage (215).

2. Appareil de diagnostic à rayons X selon la revendication 1, dans lequel
les deux sections d'opération (213, 214) disposées sur au moins un parmi le côté arrière et le côté avant de la section d'affichage (215) correspondent respectivement à une lame d'ouverture à rayons X sur un côté gauche d'une image radiographique affichée par l'appareil de diagnostic à rayons X et à une lame d'ouverture à rayons X sur un côté droit de l'image radiographique, et
les deux sections d'opération (211, 212) disposées sur au moins un parmi le côté gauche et le côté droit de la section d'affichage (215) correspondent respectivement à une lame d'ouverture à rayons X supérieure et à une lame d'ouverture à rayons X inférieure de l'image radiographique affichée par l'appareil de diagnostic à rayons X.

3. Appareil de diagnostic à rayons X selon une quelconque revendication précédente, dans lequel une taille de la première plage d'irradiation (15R) est supérieure ou égale à une taille de la seconde plage d'irradiation.

4. Appareil de diagnostic à rayons X selon la revendication 1, dans lequel
les deux sections d'opération (213, 214) disposées sur au moins un parmi le côté arrière et le côté avant de la section d'affichage (215) sont mobiles vers un côté gauche et un côté droit, et
les deux sections d'opération (211, 212) disposées sur au moins un parmi le côté gauche et le côté droit de la section d'affichage sont mobiles vers un côté arrière et un côté avant.

5. Appareil de diagnostic à rayons X selon la revendication 4,
dans lequel chacune des sections d'opération (211, 212, 213, 214) est disposée sur un rail (2110, 2120, 2130, 2140) disposé parallèlement à un côté de la première plage d'irradiation indiquée par la section d'affichage (215).

6. Appareil de diagnostic à rayons X selon la revendication 1, comprenant en outre une section de transmission (219) qui est configurée pour transmettre une sensation de clic à un opérateur par l'intermédiaire de l'ensemble d'opération (211, 212, 213, 214) lorsqu'une taille d'un champ d'irradiation atteint une taille prédéterminée.

7. Appareil de diagnostic à rayons X selon la revendication 6,
dans lequel la section de transmission (219) comprend un élément de contact (2111) qui entre en contact avec l'ensemble d'opération (211, 212, 213, 214) lorsque la taille du champ d'irradiation atteint la taille prédéterminée.

8. Appareil de diagnostic à rayons X selon la revendication 6,
dans lequel la taille prédéterminée inclut une pluralité de tailles.

9. Appareil de diagnostic à rayons X selon l'une quelconque des revendications 1 à 8,
dans lequel l'ensemble de circuits de traitement est configuré pour pouvoir commander des positions de l'ouverture à rayons X (15) de telle sorte qu'un centre de la seconde plage d'irradiation soit différent d'un centre de la première plage d'irradiation (15R), selon une opération sur la section d'opération.

10. Appareil de diagnostic à rayons X selon la revendication 1, comprenant en outre une section de désignation (218) qui est configurée pour désigner une taille prédéterminée d'un champ d'irradiation, dans lequel
l'ensemble de circuits de traitement (27) est en outre configuré pour commander des positions de l'ouverture à rayons X (15) selon la désignation de la taille du champ d'irradiation par la section de désignation (218), et pour amener l'ouverture à rayons X (15) à former la seconde plage d'irradiation correspondant au champ d'irradiation de la taille désignée par la section de désignation (218), dans lequel
l'ensemble de circuits de traitement (27) est en outre configuré pour déplacer l'ensemble d'opération (211, 212, 213, 214) vers une position correspondant à la seconde plage d'irradiation conjointement avec la formation de la seconde plage d'irradiation.

11. Appareil de diagnostic à rayons X selon la revendication 5,
les deux sections d'opération (211, 212, 213, 214) incluent des boutons présentant une relation de position dans laquelle les boutons se chevauchent dans une direction de déplacement des deux sections d'opération.

12. Procédé de fonctionnement d'un champ d'irradiation, le procédé comprenant :
la désignation, par un ensemble d'opération (211, 212, 213, 214), d'une position de chacune de quatre lames d'ouverture à rayons X (151, 152, 153, 154) incluses dans une ouverture à rayons X (15) qui limite une plage d'irradiation d'un rayon X avec lequel un sujet est irradié à partir d'un tube à rayons X (13) ;
l'indication, par une section d'affichage (215), d'une première plage d'irradiation (15R) formée par l'ouverture à rayons X (15) ; et
la commande, selon une opération sur l'ensemble d'opération (211, 212, 213, 214), d'une position d'une lame d'ouverture à rayons X (151, 152, 153, 154) correspondante et le fait d'amener l'ouverture à rayons X (15) à former une seconde plage d'irradiation,
**caractérisé en ce que**
la section d'affichage (215) est configurée séparément d'une seconde section d'affichage (23) qui affiche une image radiographique,
chacune des sections d'opération (211, 212, 213, 214) est un glisseur, et
deux des sections d'opération (213, 214) de la pluralité de sections d'opération sont disposées sur au moins un parmi un côté arrière et un côté avant de la section d'affichage (215), et deux des sections d'opération (211, 212) de la pluralité de sections d'opération sont disposées sur au moins un parmi un côté gauche et un côté droit de la section d'affichage (215).
